(19) Europäisches Patentamt
European Patent Office
Office européen des brevets

(11) EP 2 332 953 A1

(12) **EUROPEAN PATENT APPLICATION**
published in accordance with Art. 153(4) EPC

(43) Date of publication:
**15.06.2011 Bulletin 2011/24**

(21) Application number: **09811530.6**

(22) Date of filing: **27.08.2009**

(51) Int Cl.:
*C07H 19/14* (2006.01) *A61K 31/517* (2006.01)
*A61K 31/519* (2006.01) *A61K 31/7042* (2006.01)
*A61K 45/00* (2006.01) *A61K 45/06* (2006.01)
*A61P 3/06* (2006.01) *A61P 3/10* (2006.01)
*A61P 9/10* (2006.01) *A61P 13/02* (2006.01)
*A61P 13/04* (2006.01) *A61P 19/06* (2006.01)
*A61P 43/00* (2006.01) *C07D 239/70* (2006.01)

(86) International application number:
**PCT/JP2009/065376**

(87) International publication number:
**WO 2010/027005 (11.03.2010 Gazette 2010/10)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **05.09.2008 JP 2008227839**

(71) Applicant: **Kotobuki Pharmaceutical Co., Ltd.**
**Hanishina-gun, Nagano 389-0601 (JP)**

(72) Inventors:
- **TOMIYAMA Hiroshi**
**Hanishina-gun**
**Nagano 389-0601 (JP)**
- **TOKUZAKI Kazuo**
**Hanishina-gun**
**Nagano 389-0601 (JP)**
- **IWAI Yoshinori**
**Hanishina-gun**
**Nagano 389-0601 (JP)**

(74) Representative: **Albrecht, Thomas**
**Kraus & Weisert**
**Patent- und Rechtsanwälte**
**Thomas-Wimmer-Ring 15**
**80539 München (DE)**

(54) **SUBSTITUTED AMINE DERIVATIVE AND MEDICINAL COMPOSITION COMPRISING SAME AS THE ACTIVE INGREDIENT**

(57) A compound represented by following general formula (I) :

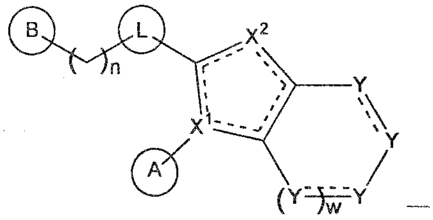

(I)

wherein, ---- represents a single bond or a double bond; L represents -NH- or the like; $X^1$ represents N or C, provided that in the case where $X^1$ is N, $X^2$ represents C-R and in the case where $X^1$ is C, $X^2$ represents N-$R^1$ or the like; Y's may be either the same or different and represent $CH_2$ or the like; A represents a non-natural sugar residue represented by following general formula:

EP 2 332 953 A1

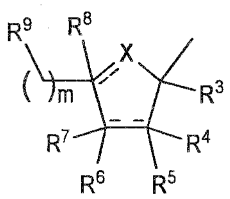

or

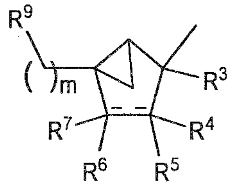

wherein, m represents 0 to 1; ---- represents a single bond or a double bond; X represents O or the like; $R^3$ to $R^8$ may be either the same or different and represent hydroxy group or the like; and B represents a group represented by following general formula:

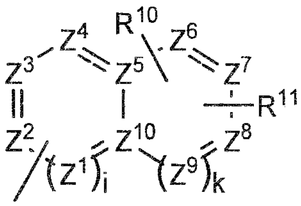

or

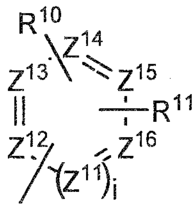

wherein, n and k represent 0 to 5; ---- represents a single bond or a double bond; $Z^1$ to $Z^{16}$ may be either the same or different and represent CH or the like; $R^{10}$ and $R^{11}$ may be either the same or different and represent a lower alkoxy group having 1 to 5 carbon atoms or the like; and $R^{15}$ represents a hydrogen atom or the like, a pharmaceutically acceptable salt thereof, a prodrug thereof, a hydrate thereof or solvate thereof. Because of having an excellent CNT2 inhibitory activity and showing a high in vivo stability and stable physicochemical properties, the aforesaid compound is useful as a remedy for hyperuricemia.

## Description

### Technical Field

This invention is concerning a new substituted amine derivative. And it is concerning a medicinal composition comprising same as an active ingredient, especially, the medicinal composition used to prevention or treatment of a disease associated with an abnormality of plasma uric acid level.

### Background Art

Gout is a purine metabolism disorder with arthropathy, nephropathy, urolithiasis and cardiovascular disorder caused by hyperuricemia and deposition of urate. And gout is idiophatic primary (gout in narrow sense) and secondary caused by inborn error of metabolism or hyperuricemia to treat malignant tumor in chemotherapy. Uric acid originates in food intake and in vivo nucleic synthesis, and it is excreted from kidney to urine as the end product. Hyperuricemia is caused by over intake of these purine bodies, synthetic enhancement of uric acid or decrease of excretion into urine. The therapeutic drugs to the hyperuricemia is divided roughly into the uric acid synthesis inhibitor which inhibits the synthesis of uric acid and uricosuric drugs which enhance the excretion of uric acid so far. Although various drugs are reported, they have still left the problem. So it is hoped to develop hyperuricemia treatment drugs with higher efficacy and few side effects. Moreover, the relation between the hyperuricemia and the metabolic syndrome is reported in recent years.

On the digestion and absorption pathway of nucleic acid in human, nucleic acid and nucleoproteins which are released in the intestine from food intake are broken down into mononucleotides by ribonucleases, deoxyribonucleases and polynucleotidases. Furthermore mononucleotide is degraded into nucleoside by nucleotidases and phosphateses and then the nucleosides are absorbed by membrane proteins called nucleoside transporter. In regard with the study of nucleoside transporter, it is reported that purinenucleosides are absorbed by a subtype called CNT2 which is expressed mainly in small intestine(Non Patent Literature 1). According to this report, the absorption of the purincnucleoside in small intestines could be obstructed by inhibiting CNT2, and therefore that could treat hyperuricemia by decreasing plasma uric acid levels. Drug which obstructs CNT2, that is, the CNT2 inhibitors are proposed based on this idea. And, the compound shown by the general type of following chemical formula 1, chemical formula 2, chemical formula 3, and chemical formula 4 is proposed as CNT2 inhibitors(Patent Literature 1 ~ 4).

[Chemical formula 1]

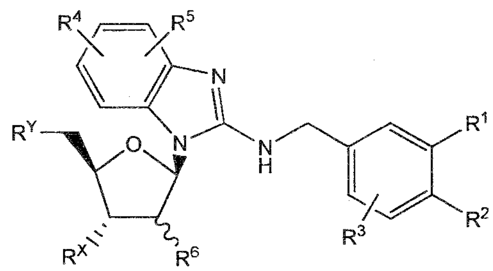

[Chemical formula 2]

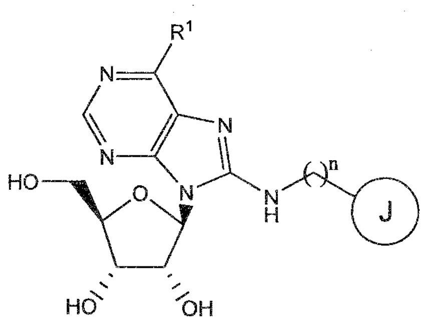

[Chemical formula 3]

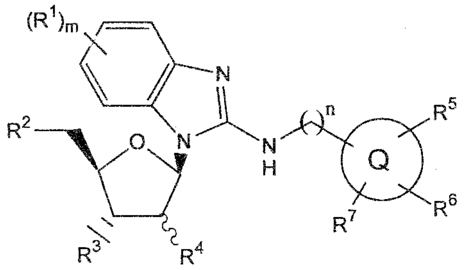

[Chemical formula 4]

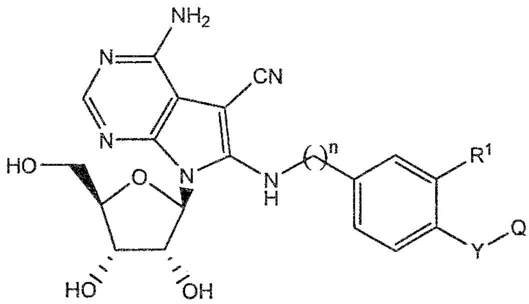

Although these compounds are showed uric acid control action, the activity of them is not satisfying and neither stability in physiological condition nor the physicochemical stability are enough.

Citation List

Patent Literature 1 : International publication no.WO2005/06378 pamphlet.
Patent Literature 2 : International publication no.WO2006/030803 pamphlet.
Patent Literature 3 : International publication no.WO2006/115137 pamphlet.
Patent Literature 4 : International publication no.WO2007/277203 pamphlet.
Non Patent Literature 1 : Ho-Chul Shin, Jin-Suk Kim, Balvinder Singh Vig, Xueqin Song, John Charles Drach and Gordon Lewis Amidon, Biol. Pharm. Bull. 29, (2) 247.

Summary of Invention

Technical Problem

This invention aims to offer the compound that shows an excellent inhibitory activity, a good stability in physiological condition, and an excellent physicochemical stability. Moreover, it aims to develop hyperuricemia treatment drugs with higher efficacy and few side effect by using this inhibitory activity.

Solution to Problem

This inventor was thought that the compound of Patent Literature 1~4 that had the ribose as a sugar recidue was physically unstable and was unstable against ribonuclease or deoxyribonuclease exist in intestines, and were examined the various compounds that showed an excellent inhibitory activity and good stability. As a result, the compound represented by the formula (I) was found that showing the excellent CNT2 inhibitory activity and suppressing the systemic absorption of the purinenucleoside, and this invention was completed.

Namely, the compounds of the present invention have the following general formula (I) :

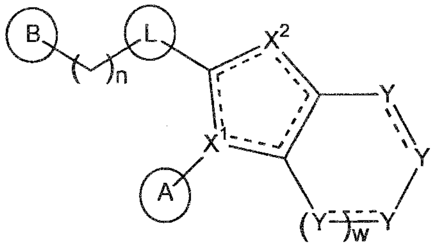

--- (I)

wherein,

n and w represent 0 to 5;

---- represents a single bond or a double bond;

$X^1$ represents N or C, provided that in the case where $X^1$ is N, $X^2$ represents C-R and in the case where $X^1$ is C, $X^2$ represents N-$R^1$, $CR^1R^2$, O or S;

wherein,

R represents hydrogen atom, lower alkyl group having one to five carbon atoms, lower alkyl group including cycloalkyl ring having three to five carbon atoms, lower alkenyl group having two to five carbon atoms, lower alkynyl group having two to five carbon atoms, lower haloalkyl group having one to five carbon atoms, lower alkoxyalkyl group having two to five carbon atoms, lower hydroxyalkyl group having one to five carbon atoms, lower alkoxycarbonyl group having two to five carbon atoms, lower aminoalkyl group having one to five carbon atoms, hydroxy group, amino group, mercapto group, carboxyl group, carbamoyl group, alkanoyl group, cyano group or halogen group;

$R^1$ and $R^2$ may be either the same or different and represent hydrogen atom, lower alkyl group having one to five carbon atoms or lower haloalkyl group having one to five carbon atoms;

L represents N-$R^1$, $CR^1R^2$, lower alkenylene group having two to five carbon atoms, lower alkynylene group having two to five carbon atoms or $S(O)_v$;

wherein,

$R^1$ and $R^2$ are same meaning as defined in the above;

v represents 0 to 2;

Y's may be either the same or different and represent $CH_2$, CH, O, S, N-$R^1$, CH-$NH_2$, CH-OH, CH-SH, CH-halogen atom, C-$NH_2$, C-OH, C-SH or C-halogen atom;

A represents the following general formula;

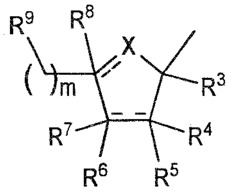

or

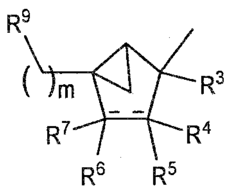

wherein,

m represents 0 to 1;

--- represents a single bond or a double bond;

X represents CH, $CH_2$. O, $S(O)_v$, N-$R^1$, $CF_2$ or -(=$CH_2$)-;

$R^3$, $R^4$, $R^5$, $R^6$, $R^7$, $R^8$ and $R^9$ may be either the same or different and represent hydrogen atom, hydroxyl group, lower alkylamino group having one to five carbon atoms, mercapto group , -$(CH_2)_u$-lower alkoxy group having one to five carbon atoms, halogen atom, lower haloalkyl group having one to five carbon atoms, lower alkyl group having one to five carbon atoms, lower alkyl group including cycloalkyl ring having three to five carbon atoms, lower alkenyl group having two to five carbon atoms or lower alkynyl group having two to five carbon atoms;

$R^3$, $R^4$, $R^5$, $R^6$, $R^7$, $R^8$ and $R^9$ optionally formed the cyclo or the heterocyclo ring;

u represents 0 to 5;

however, in the general formula (I), in the case where $X^1$ is N, $X^2$ is C-CN, X is O, --- is a single bond and $R^5$, $R^6$ and $R^9$ are hydroxy group, $R^4$ is not hydrogen atom;

B represents the following general formula;

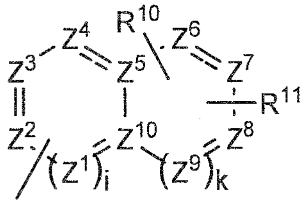

or

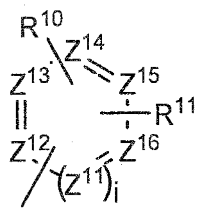

wherein,

i and k represent 0 to 5;

---- represents a single bond or a double bond;

$Z^1$, $Z^2$, $Z^3$, $Z^4$, $Z^5$, $Z^6$, $Z^7$, $Z^8$, $Z^9$, $Z^{10}$, $Z^{11}$, $Z^{12}$, $Z^{13}$, $Z^{14}$, $Z^{15}$ and $Z^{16}$ may be either the same or different and represent CH, $CH_2$, O, S, N or NH;

$R^{10}$ and $R^{11}$ may be either the same or different and represent hydrogen atom, halogen atom, lower alkyl group having one to five carbon atoms, optionally substituted cycloalkyl group having three to eight carbon atoms, optionally substituted heterocycloalkyl group, lower haloalkyl group having one to five carbon atoms, lower alkenyl group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, nitro group, cyano group, optionally substituted mono or bicyclic aromatic ring having five to ten carbon atoms, optionally substituted mono or bicyclic heteroaromatic ring having one to ten carbon atoms, optionally substituted benzyl group, optionally substituted amino group(optionally formed a quaternary ammonium compound), $-CONR^{12}R^{13}$, $-NHCOR^{15}$, $-(CH_2)_o-R^{15}-(CH_2)_p-R^{16}$, -lower alkenylene group having two to five carbon atoms-$R^{15}$, -lower alkynylene group having two to five carbon atoms-$R^{15}$, $-(CH_2)_o$-cycloalkyl group having three to eight carbon atoms-$(CH_2)_p-R^{15}$, $-(CH_2)_o$-heterocycloalkyl group-$(CH_2)_p-R^{15}$, $-(CH_2)_o-NHC(=NH)-NH_2$, $-(CH_2)_o-C(=NH)-NH_2$, $-(CH_2)_o-D-(CH_2)_t-E-(CH_2)_p-R^{15}$ or a group represented by the following general formula;

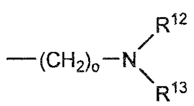 , 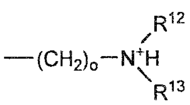 , 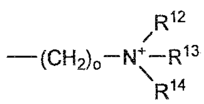 , 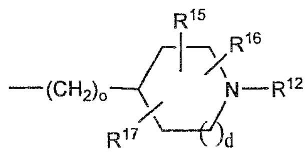 ,

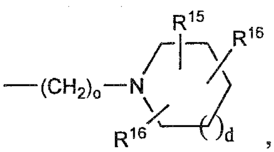 , 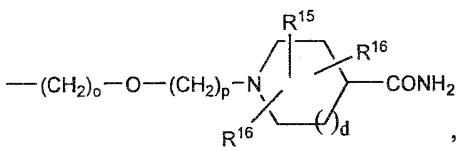 , 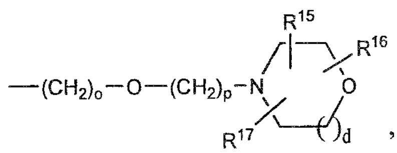 ,

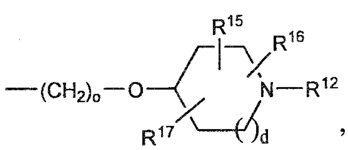 , 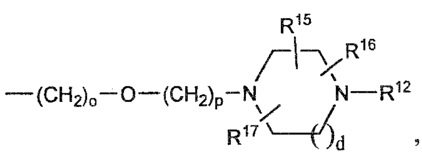 , 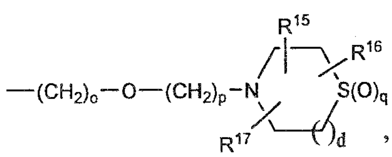 ,

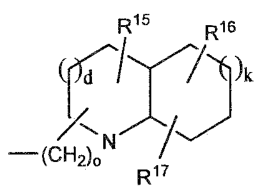 or 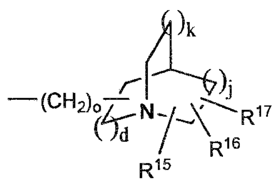

wherein,

o and p may be either the same or different and represent 0 to 15;

t represents 1 to 5;
q represents 0 to 2;
d, k and j represent 0 to 5;
D and E may be either the same or different and represent single bond(-), $-CONR^{12}-$, $-NR^{22}CO-$, -O $-NR^{22}-$, $-S(O)_q-$, -N $R^{12}$ $S(O)_q-$, $-S(O)_qNR^{12}-$ or -NHC(=O)NH-;
$R^{12}$, $R^{13}$ and $R^{14}$ may be either the same or different and represent hydrogen atom, carboxyl group, lower alkyl group having one to five carbon atoms or lower hydroxy alkyl group having one to five carbon atoms and optionally formed the ring;
$R^{15}$, $R^{16}$ and $R^{17}$ represent hydrogen atom, halogen atom, lower alkyl group having one to five carbon atoms, optionally substituted cycloalkyl group having three to eight carbon atoms, optionally substituted heterocycloalkyl group, lower haloalkynylene group having two to five carbon atoms, lower alkenylene group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, sulfonic acid group, phosphoric acid group, nitro group, cyano group, aminosulfonyl group, optionally substituted mono or bicyclic aromatic ring having five to ten carbon atoms, optionally substituted mono or bicyclic heteroaromatic ring having one to ten carbon atoms, optionally substituted benzyl group, -S(O)q-lower alkyl group having one to five carbon atoms, $-CONR^{18}R^{19}$, optionally substituted amino group(optionally formed a quaternary ammonium compound), amino acid residue, sugar residue or a group represented by the following general formula;

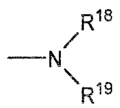 , 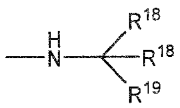 ,

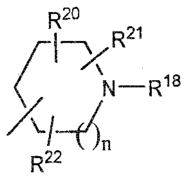 , 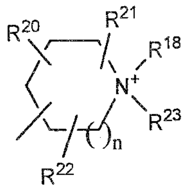 or 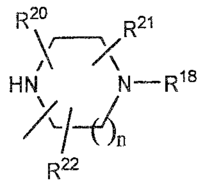

wherein,
$R^{18}$ and $R^{19}$ represent hydrogen atom, lower alkyl group having one to five carbon atoms, lower alkylcarbonyl group having two to five carbon atoms, lower alkylsulfonyl group having one to five carbon atoms, optionally substituted benzenesulfonyl group, lower alkoxycarbonyl group having two to five carbon atoms, lower hydroxyalkyl group having one to five carbon atoms, hydroxyethoxy group, alkoxyethoxy group, optionally substituted aminoethoxy group or $-(CH_2)_o-CONH_2$;
$R^{20}$, $R^{21}$ and $R^{22}$ represent hydrogen atom, halogen atom, lower alkyl group having one to five carbon atoms, optionally substituted cycloalkyl group having three to eight carbon atoms, optionally substituted heterocycloalkyl group, lower haloalkynylene group having two to five carbon atoms, lower alkenylene group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, nitro group, cyano group, aminosulfonyl group, optionally substituted mono or bicyclic aromatic ring having five to ten carbon atoms, optionally substituted mono or bicyclic heteroaromatic ring having one to ten carbon atoms, optionally substituted benzyl group, $-S(O)_q$-lower alkyl group having one to five carbon atoms, optionally substituted amino group(optionally formed a quaternary ammonium compound);
$R^{23}$ represents lower alkyl group having one to five carbon atoms;
or a pharmaceutically acceptable salt thereof, or a prodrug thereof, or a hydrate or solvate thereof.

Namely, the compounds as defined in the above general formula (I) represented by the following general formula (II) ~ (IV):

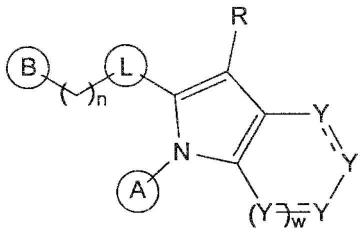

--- (II)

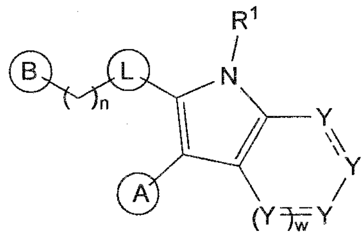

--- (III)

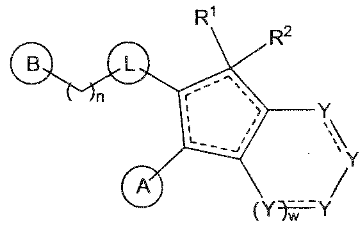

--- (IV)

wherein,
A, B, L, R, $R^1$, $R^2$ and Y are same meaning as defined in the above;
n and w represent 0 to 5;
---- represents a single bond or a double bond;
or a pharmaceutically acceptable salt thereof, or a prodrug thereof, or a hydrate or solvate thereof.

In the compounds as defined in the above general formula (I), it is desirable that A is the substituent represented by the following general formula:

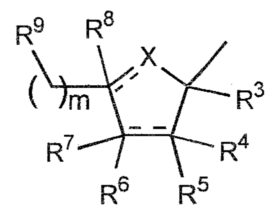

wherein,
X represents O;
--- represents a single bond or a double bond;
$R^3$, $R^4$, $R^6$, $R^7$ and $R^8$ may be either the same or different and represent hydrogen atom, hydroxy group, amino group, mercapto group, $-(CH_2)_u$-lower alkoxy group having one to five carbon atoms, halogen atom, lower haloalkyl group having one to five carbon atoms, lower alkyl group having one to five carbon atoms, lower alkenyl group having two to five carbon atoms or lower alkynyl group having two to five carbon atoms;

u represents 0 to 5;
$R^5$ represents hydroxy group, halogen atom, lower haloalkyl group having one to five carbon atoms, lower alkyl group having one to five carbon atoms, lower alkenyl group having two to five carbon atoms or lower alkynyl group having two to five carbon atoms;
$R^9$ represents hydroxy group or halogen atom;
$R^3$, $R^4$, $R^5$, $R^6$, $R^7$, $R^8$ and $R^9$ optionally formed the ring;
or a pharmaceutically acceptable salt thereof, or a prodrug thereof, or a hydrate or solvate thereof.

And in the compounds as defined in the above general formula (I), it is desirable that A is the substituent represented by the following general formula:

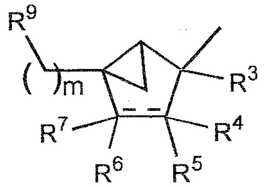

wherein,
--- represents a single bond or a double bond;
$R^3$, $R^4$, $R^5$, $R^6$ and $R^7$ may be either the same or different and represent hydrogen atom, hydroxy group, amino group, mercapto group, $-(CH_2)_u$-lower alkoxy group having one to five carbon atoms, halogen atom, lower haloalkyl group having one to five carbon atoms, lower alkyl group having one to five carbon atoms, lower alkenyl group having two to five carbon atoms or lower alkynyl group having two to five carbon atoms;
$R^9$ represent hydroxy group or halogen atom;
u represents 0 to 5;
$R^3$, $R^4$, $R^5$, $R^6$, $R^7$ and $R^9$ optionally formed the ring;
or a pharmaceutically acceptable salt thereof, or a prodrug thereof, or a hydrate or solvate thereof.

Moreover, in the compoumds as defined in above general formula (I), it is desirable that A is a five-membered ring that can take the dominant Northem-lock comformation, or a pharmaceutically acceptable salt thereof, or a prodrug thereof, or a hydrate or solvate thereof. In the compoumds as defined in above general formula (I), it is desirable that the compound wherein L is N-$R^1$ and $R^1$ is hydrogen atom, or a pharmaceutically acceptable salt thereof, or a prodrug thereof, or a hydrate or solvate thereof.

This invention is concerning a pharmaceutical composition comprising as an active ingredient a compound as defined in the above, or a pharmaceutically acceptable salt thereof, or a prodrug thereof, or a hydrate or solvate thereof. A pharmaceutical composition as defined in the above, which is an agent for the prevention or treatment of a disease associated with an abnormality of plasma uric acid level. Wherein the disease associated with an abnormality of plasma uric acid level is a disease selected from a group consisting of gout, hyperuricemia, urinary lithiasis, hyperuricemic nephropathy and acute uric acid nephropathy. Moreover, this invention is concerning a pharmaceutical composition as defined in the above comprising in combination as an active ingredient at least one agent selected from a group consisting of colchicine, a nonsteroidal anti-inflammatory agent, a steroid, a uric acid synthesis inhibitor, a uricosuric drug, a urinary alkalinizer and a uric acid oxidase.

1. Furthermore, this invention is concerning a pharmaceutical composition as defined in the above comprising in combination as an active ingredient at least one metabolic syndrome agent selected from a group consisting of an antihypertensive drug, an antihyperglycemic drug, an antiobestic drug, a nucleic acid metabolism inhibitor, a diuretic, an antituberculous drug, an antiinflammatory drug, an antihyperlipidemic drug, an antiasthmatic drug, a cholesterol synthesis inhibitor, a cholesterol absorption inhibitor, an immunosuppressive drug, an agent of abnormality of intravascular pressure, an ischemic heart disease agent, a β - 3 adrenoceptor agonist, a cannabinoid agonist or antagonist, a neuropeptide Y5 receptor antagonist , an apo-B/MTP inhibitor, a 11-β hydroxysteroid dehydrogcnase-1 inhibitor, a peptideYY$_{3-36}$ agonist, a MCR4 agonist, a CCK-A agonist, a monoamine reuptake inhibitor, a sympathetic agent, a dopamine agonist, a melanocyte-stimulating hormone receptor agonist, a leptin receptor agonist, a galanin receptor antagonist, a thyroid stimulator, a glucocorticoid receptor antagonist, an orexin receptor antagonist, an epiandrosterone dehydrogenase agent, a H-3 receptor antagonist, a lipase inhibitor, a PPAR ligand modulator, a insulinotropic agent, a sulfonylurea, a α-glucosidase inhibitor, an insulin-sensitizing drug, a non-thiazolidinone compound, a protein tyrosine phosphatase-B inhibitor, a dipeptidyl peptidase-IV inhibitor, a glucagon antagonist, a HMG-CoA reductase inhibitor, a sterol ester, an ACAT inhibitor, a bile acid reuptake inhibitor, a microsome triglyceride transporter inhibitor,

a fibric acid derivatives, a β-blocker, an ACE inhibitor, a calcium channel blocker, a renin inhibitor, an AT- 1 receptor antagonist, an ET receptor antagonist and an AII receptor antagonist.

Advantageous Effects of Invention

The compound represented by the general formula (I) of the present invention was found that showing the stability to the hydrolysis, the physicochemical stability and the good CNT2 inhibitory activity. Especially, the compound that fixed the conformation of the furanose ring to Northern has greatly improved the stability to the hydrolysis, the physicochemical stability and the CNT2 inhibitory activity. The compounds represented by the general formula (I) of the present invention possess the excellent CNT2 inhibitory activity and suppress the systemic absorption of the purinenucleoside, therefore, they are useful as agents for the decreased of plasma uric acid level.

Brief Description of Drawings

Fig.1 is a graph where the result of Test Example 3 is shown.

Description of Embodiments

The compound represented by the general formula (I) of the present invention is described in detail.
$X^1$ represents N or C, provided that in the case where $X^1$ is N, $X^2$ represents C-R and in the case where $X^1$ is C, $X^2$ represents N-$R^1$, $CR^1R^2$, O or S;
wherein,
R represents a hydrogen atom, lower alkyl group having one to five carbon atoms, lower alkyl group including cycloalkyl ring having three to five carbon atoms, lower haloalkyl group having one to five carbon atoms, lower alkoxyalkyl group having two to five carbon atoms, lower hydroxyalkyl group having one to five carbon atoms, lower alkoxycarbonyl group having two to five carbon atoms, lower aminoalkyl group having one to five carbon atoms, hydroxy group, amino group, mercapto group, carboxyl group, carbamoyl group, alkanoyl group, cyano group or halogen group;
$R^1$ and $R^2$ may be either the same or different and represent a hydrogen atom, lower alkyl group having one to five carbon atoms or lower haloalkyl group having one to five carbon atoms;
a number of carbons here is number of carbons all the substitution group;
L represents N-$R^1$, $CR^1R^2$, optionally branched alkenylene group(for example, -(CH=CH)-, $-CH_2$(CH=CH)-, -(CH=CH) $CH_2$-, $-(CH_2)_2$(CH=CH)-, -(CH=CH)$(CH_2)_2$-, $-(CHCH_3)$(CH=CH)-, -(CH=CH)$(CHCH_3)$- or the like), optionally branched alkynylene group(for example, ethylene group, propene group, isopropene group, butene group, isobutene group, pentene group, isopentene group, 2,2-dimethylpropene group or the like) or $S(O)_v$ (for example, sulfide group, sulfoxide group or sulfone group);
Y's may be either the same or different and represent $CH_2$, CH, O, S, N-$R^1$, $CH-NH_2$, CH-OH, CH-SH, CH-halogen atom, $C-NH_2$, C-OH, C-SH, C-halogen atom;
A represents the following general formula;

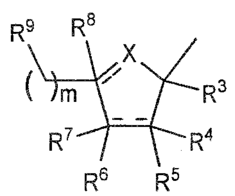

or

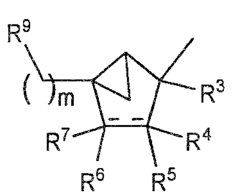

wherein, it is known that the five membered ring that has the frame of the present A takes the many conformations. All these conformations are shown as angle P on the pseuderotational cycle. As an example of the representative, the conformation of P=0°, P=90°, P=180° and P=270° is called the Northern, Eastern, Southern and Western, respectively (C. Altona, M. J. Sundaralingham, J. Am. Chem. Soc.,1972, 94, 8205.).
The following formula is an example of the ribose. Wherein, Ar represents a purine or pyrimidine.

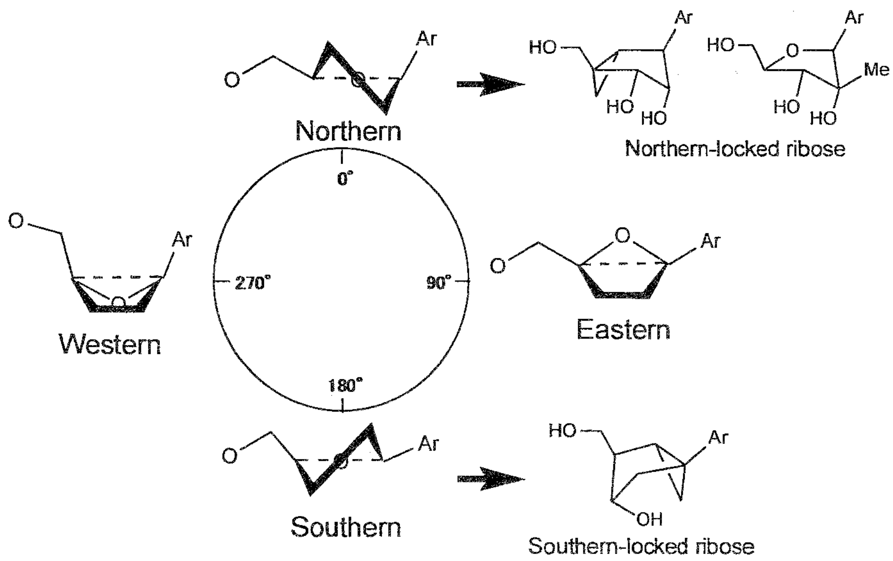


The compound with dominant conformation of Northern and Southern is reported in the field such as the antiviral drugs (Jonathan K. Watts, Kashinath Sadalapure, Niloufar Choubdar, B. Mario Pinto, Masad J. Damha, J. Org. Chem., 2006, 71, 921., Victor E. Marquez, Maqbool A. Siddiqui, Abdallah Ezzitouni, Pamela Russ, Jianying Wang, Richard W. Wagner, Mark D. Matteucci, J. Med. Chem., 1996, 39, 3739.). However, the compound that fixed the stability conformation of the nucleoside is not reported in the field of CNT2 inhibitor. It was difficult to expect which conformation of the Northern, Eastern, Southern and Western were active configuration, but the compound of the Northem-lock conformation greatly improved the CNT2 inhibitory activity, the stability to the hydrolysis and the physicochemical stability compared with the compound known so far.

In the above formula A, $R^3$, $R^4$, $R^5$, $R^6$, $R^7$ and $R^8$ may be either the same or different and represent hydrogen atom, hydroxyl group, $-(CH_2)_u$-lower alkoxy group(for example, methoxy group, ethoxy group, propoxy group, isopropoxy group, butoxy group, isobutoxy group or the like), halogen atom(for example, fluorine atom, chlorine atom, bromine atom, iodine atom), lower alkyl group(for example, methyl group, ethyl group, propyl group, isopropyl group, butyl group, isobutyl group, sec-butyl group, tert-butyl group, pentyl group, neopentyl group or the like), cycloalkyl group(for example, cyclopropyl group, cyclobutyl group, cyclopentyl group or the like), lower haloalkyl group(for example, trifluoromethyl group, 2,2,2-trifluoroethyl group, 4-bromobutyl group, 5-chloropropyl group or the like), lower alkenyl group(for example, ethylene group, propenyl group, isopropenyl group, butenyl group or the like), lower alkynyl group(for example, ethynyl group, propargyl group or the like). $R^9$ represents hydroxy group, halogen atom(for example, fluorine atom, chlorine atom, bromine atom, iodine atom).

In the above formula B, $R^1$ and $R^{11}$ may be either the same or different and represent hydrogen atom, halogen atom(for example, fluorine atom, chlorine atom, bromine atom, iodine atom), lower alkyl group(for example, methyl group, ethyl group, propyl group, isopropyl group, butyl group, isobutyl group, sec-butyl group, tert-butyl group, pentyl group, neopentyl group or the like), cycloalkyl group(for example, cyclopropyl group, cyclobutyl group, cyclopentyl group or the like), heterocycloalkyl group(for example, morpholine, piperidine, piperazine, tetrahydrofuran, dioxane, dithiane or the like), lower haloalkyl group(for example, trifluoromethyl group, 2,2,2-trifluoroethyl group or the like), lower alkenyl group(for example, ethylene group, propenyl group, isopropenyl group, butenyl group or the like), lower alkynyl group (for example, ethynyl group, propargyl group or the like), hydroxy group, optionally substituted amino group(for example, amino group, methylamino group, dimethylamino group, ethylamino group, diethylamino group, propylamino group, dipropylamino group, isopropylamino group, diisopropylamino group, acetylamino group, methanesulfonylamino group, methoxycarbonylamino group, t-butoxycarbonyl- amino group or the like), lower alkoxy group(for example, methoxy group, ethoxy group, propoxy group, isopropoxy group, butoxy group, isobutoxy group or the like), lower haloalkoxy group(for example, chloromethoxy group, bromomethoxy group, dichloromethoxy group, iodopropoxy group, 4,4-difluorobutoxy group or the like), lower alkoxycarbonyl group(for example, methoxycarbonyl group, ethoxycarbonyl group, propoxycarbonyl group, isopropoxycarbonyl group or the like), carboxyl group, nitro group, cyano group, optionally substituted mono or bicyclic aromatic ring(for example, phenyl group, fluorophenyl group, chlorophenyl group, methylphenyl group, methoxyphenyl group, aminophenyl group, cyanophenyl group, methylthiophenyl group, methanesulfonylphenyl group, naphthalene ring, azulene ring or the like), optionally substituted mono or bicyclic heteroaromatic ring (for example, pyridine ring, furan ring, thiophene ring, imidazole ring, thiazole ring, benzofuran ring, benzothiophene ring, benzoimidazole ring, benzothiazole ring or the like).

$R^{12}$, $R^{13}$ and $R^{14}$ may be either the same or different and represent hydrogen atom, carbonyl group, carboxyl group, optionally cyclized lower alkyl group(for example, methyl group, ethyl group, propyl group, isopropyl group, butyl group, isobutyl group, sec-butyl group, tert-butyl group, pentyl group, neopentyl group or the like).

$R^{15}$, $R^{16}$ and $R^{17}$ represent hydrogen group, halogen atom(for example, fluorine atom, chlorine atom, bromine atom, iodine atom), lower alkyl group(for example, methyl group, ethyl group, propyl group, isopropyl group, butyl group, isobutyl group, sec-butyl group, tert-butyl group, pentyl group, neopentyl group or the like), cycloalkyl group(for example, cyclopropyl group, cyclobutyl group, cyclopentyl group or the like), heterocycloalkyl group(for example, morpholine, piperidine, piperazine, tetrahydrofuran, dioxane, dithiane or the like), lower haloalkyl group(for example, trifluoromethyl group, 2,2,2-trifluoroethyl group or the like), lower alkenyl group(for example, ethylene group, propenyl group, isopropenyl group, butenyl group or the like), lower alkynyl group(for example, ethynyl group, propargyl group or the like), hydroxy group, optionally substituted amino group(for example, amino group, methylamino group, dimethylamino group, ethylamino group, diethylamino group, propylamino group, dipropylamino group, isopropylamino group, diisopropylamino group, acetylamino group, methanesulfonylamino group, methoxycarbonylamino group, t-butoxycarbonylamino group or the like), lower alkoxy group(for example, methoxy group, ethoxy group, propoxy group, isopropoxy group, butoxy group, pentyloxy group, cyclopentyloxy or the like), lower haloalkoxy group(for example, trifluoromethoxy group, 2,2,2-trifluoroethoxy group or the like), lower alkoxycarbonyl group(for example, methoxycarbonyl group, ethoxycarbonyl group, propoxycarbonyl group, isopropoxycarbonyl group or the like), carboxyl group, sulfonic acid group, phosphoric acid group, nitro group, cyano group, aminosulfonyl group, optionally substituted mono or bicyclic aromatic ring(for example, phenyl group, fluorophenyl group, chlorophenyl group, methylphenyl group, methoxyphenyl group, aminophenyl group, cyanophenyl group, methylthiophenyl group, methanesulfonylphenyl group, naphthalene ring, azulene ring or the like), optionally substituted mono or bicyclic heteroaromatic ring(for example, pyridine ring, furan ring, thiophene ring, imidazole ring, thiazole ring, benzofuran ring, benzothiophene ring, benzoimidazole ring, benzothiazole ring or the like), optionally substituted benzyl group(for example, chlorobenzyl group, bromobenzyl group, nitrobenzyl group, ethylbenzyl group, isopropylbenzyl group or the like), $-S(O)_q$-lower alkyl group(for example, methyl group, ethyl group, propyl group, isopropyl group, butyl group, pentyl group, cyclopentyl group or the like), $-CONR^{17}R^{18}$, optionally substituted amino group(for example, amino group, methylamino group, dimethylamino group, ethylamino group, diethylamino group, propylamino group, dipropylamino group, isopropylamino group, diisopropylamino group, acetylamino group, methanesulfonylamino group, methoxycarbonylamino group, t-butoxycarbonylamino group or the like), amino acid residue(for example, glycine, alanine, valine, leucine, isoleucine, phenylalanine, asparagine, glutamine, tryptophan, proline, serine, threonine, tyrosine, cysteine, cystine, methionine, aspartic acid, glutamic acid, lysine, arginine, histidine, 2-aminoadipic acid, 2-aminoisobutyric acid, 3-aminoadipic acid, 3-aminoisobutyric acid, β-alanine, 2-aminopimelic acid, 2-aminobutyric acid, 2,4-diaminobutyric acid, 4-aminobutyric acid, desmosine, piperidinecarboxylic acid, 2,2-diaminopimelic acid, 6-aminocaproic acid, 2,3-diaminopropionic acid, 2-aminoheptanoic acid, N-ethyl-glycine, 2-(2-thienyl) glycine, 3-(2-thienyl)alanine, penicillamine, sarcosine, N-ethyl-asparagine, N-methylisoleucine, hydroxylysine, 6-N-methyllysine, Allo-hydroxylysine, N-methylvaline, 3-hydroxyproline, norvaline, 4-hydroxyproline, norleucine, iso-desmosine, ornithine, Allo-isoleucine and its derivatives or the like), sugar residue (for example, the O-glycoside, N-glycoside, C-glycoside or the like of glucose, mannose, fructose, galactose, ribose, erythrose, glyceraldehyde, sedoheptulose, glucosamine, galactosamine, glucuronic acid, galactonic acid, mannonic acid, glucamine, 3-amino-1,2-propanediol, glucaric acid, galactaric acid etc. including amino sugar, amino alcohol and sugar acid derived from aldose and ketose).

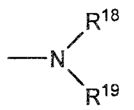 , 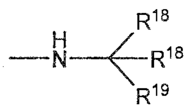 ,

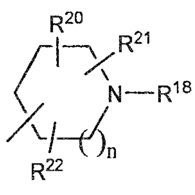 , 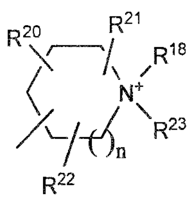 or 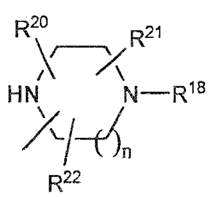

$R^{18}$ and $R^{19}$ represent hydrogen atom, lower alkyl group(for example, methyl group, ethyl group, propyl group, isopropyl group, butyl group, pentyl group, cyclopentyl group or the like), lower alkylcarbonyl group(for example, methylcarbonyl group, ethylcarbonyl group, propylcarbonyl group, butylcarbonyl group, pentylcarbonyl group or the like), lower alkylsulfonyl group(for example, methanesulfonyl group, ethanesulfonyl group, propanesulfonyl group, butanesulfonyl group, pentanesulfonyl group or the like), optionally substituted benzenesulfonyl group(for example, methylbenzenesulfonyl group, ethylbenzenesulfonyl group, chlorobenzenesulfonyl group, bromobenzenesulfonyl group or the like), lower alkoxycarbonyl group(for example, methoxycarbonyl group, ethoxycarbonyl group, propoxycarbonyl group, isopropoxycarbonyl group, butoxycarbonyl group, pentoxycarbonyl group or the like), hydroxyethoxy group, alkoxyethoxy group(for example, methoxyethoxy group, ethoxyethoxy group, propoxyethoxy group, butoxyethoxy group, pentoxyethoxy group or the like), optionally substituted aminoethoxy group(for example, aminoethoxy group, methylaminoethoxy group, dimethylaminoethoxy group, ethylaminoethoxy group, propylaminoethoxy group, butylaminoethoxy group, pentylaminoethoxy group or the like), $-(CH_2)_o-CONH_2$.

$R^{20}$, $R^{21}$ and $R^{22}$ represent hydrogen atom, halogen atom(for example, fluorine atom, chlorine atom, bromine atom, iodine atom), lower alkyl group(for example, methyl group, ethyl group, propyl group, isopropyl group, butyl group, isobutyl group, sec-butyl group, tert-butyl group, pentyl group, neopentyl group or the like), cycloalkyl group(for example, cyclopropyl group, cyclobutyl group, cyclopentyl group or the like), heterocycloalkyl group(for example, morpholine, piperidine, piperazine, tetrahydrofuran, dioxane, dithiane or the like), lower haloalkyl group(for example, trifluoromethyl group, 2,2,2-trifluoroethyl group or the like), lower alkenyl group(for example, ethylene group, propenyl group, isopropenyl group, butenyl group or the like), lower alkynyl group(for example, ethynyl group, propargyl group or the like), hydroxy group, optionally substituted amino group(for example, amino group, methylamino group, dimethylamino group, ethylamino group, diethylamino group, propylamino group, dipropylamino group, isopropylamino group, diisopropylamino group, acetylamino group, methanesulfonylamino group, methoxycarbonylamino group, t-butoxycarbonylamino group or the like), lower alkoxy group(for example, methoxy group, ethoxy group, propoxy group, isopropoxy group, butoxy group, pentyloxy group, cyclopentyloxy group or the like), lower haloalkoxy group(for example, trifluoromethoxy group, 2,2,2-trifluoroethoxy group or the like), lower alkoxycarbonyl group(for example, methoxycarbonyl group, ethoxycarbonyl group, propoxycarbonyl group, isopropoxycarbonyl group or the like), carboxyl group, nitro group, cyano group, optionally substituted mono or bicyclic aromatic ring(for example, phenyl group, fluorophenyl group, chlorophenyl group, methylphenyl group, methoxyphenyl group, aminophenyl group, cyanophenyl group, methylthiophenyl group, methanesulfonylphenyl group, naphthalene ring, azulene ring or the like), optionally substituted mono or bicyclic heteroaromatic ring (for example, pyridine ring, furan ring, thiophene ring, imidazole ring, thiazole ring, benzofuran ring, benzothiophene ring, benzoimidazole ring, benzothiazole ring or the like), optionally substituted benzyl group(for example, chlorobenzyl group, bromobenzyl group, nitrobenzyl group, ethylbenzyl group, isopropylbenzyl group or the like), $-S(O)_q$-lower alkyl group(for example, methyl group, ethyl group, propyl group, isopropyl group, butyl group, pentyl group, cyclopentyl group or the like), optionally substituted amino group(for example, amino group, methylamino group, dimethylamino group, ethylamino group, diethylamino group, propylamino group, dipropylamino group, isopropylamino group, diisopropylamino group, acetylamino group, methanesulfonylamino group, methoxycarbonylamino group, t-butoxycarbonylamino group or the like).

$R^{22}$ represents lower alkyl group(for example, methyl group, ethyl group, propyl group, isopropyl group, butyl group, pentyl group, cyclopentyl group or the like).

$R^{24}$ represents hydrogen atom, lower alkyl group(for example, methyl group, ethyl group, propyl group, isopropyl group, butyl group, pentyl group, cyclopentyl group or the like) or lower haloalkyl group(for example, trifluoromethyl group, 2,2,2-trifluoroethyl group, 4-bromobutyl group, 5-chloropropyl group or the like).

This invention is concerning a compound represented by general formula (I), or a pharmaceutically acceptable salt thereof, or a prodrug thereof, or a hydrate or solvate thereof. The compound of this invention is containing any stereoisomers, optical isomers, tautomers, and these arbitrary mixture or the like.

"Their pharmaceutically acceptable salts" maintain the biological efficacy and the character of this inventive compound and indicate an addition salts that dose not give the inconvenience in biologically or other characters. The addition salts of this invention compound include the ones described in WILEY-VCH published "Handbook of Pharmaceutical Salts (2002)". Examples of such salts include acid addition salts such as hydrochloric acid, hydrobromic acid, hydroiodic acid, sulfuric acid, nitric acid, phosphoric acid, methanesulfonic acid, benzenesulfonic acid, p-toluenesulfonic acid, formic acid, acetic acid, trifluoroacetic acid, oxalic acid, citric acid, malonic acid, fumaric acid, glutaric acid, adipic acid, maleic acid, tartaric acid, succinic acid, mandelic acid, malic acid, pantothenic acid, glutamic acid, aspartic acid or the like, and base addition salts such as lithium hydroxide, potassium hydroxide, sodium hydroxide, calcium hydroxide, magnesium hydroxide, ammonia, isopropylamine, diethylamine, triethylamine, ethanolamine, piperidine, pyridine, tris (hydroxymethyl) methylamine, tris(hydroxyethyl) methylamine, lysine, arginine, choline or the like.

"Their prodrug" indicate the compound converted into this inventive compound by the reaction with the enzyme, the gastric juice or the like under the physiological condition. For example, it indicates the compound converted into this inventive compound by the oxidizing reaction, the reductive reaction and the hydrolytic reaction caused by the enzyme and the hydrolytic reaction caused by the gastric acid or the like. The groups that form this prodrug include the groups described in Prog. Med., 5, 2157, 1985. or the like.

"Their hydrates" indicate the hydrate formed by a this inventive compound or a pharmaceutically acceptable salt thereof absorbed moisture. And "their solvates" indicate what became a solvate, when this inventive compound is

left in the solvent and the recrystallized.

The compound represented by the general formula (I) is concretely illustrated as follows.

( 1 ) 4-Amino-6-[(biphenyl-4-ylmethyl)-amino]-7-(3,4-dihydroxy-5-hydroxymethyl-3-methyl-tetrahydro-furan-2-yl)-7H-pyrrolo[2,3-d]pyrimidine-5-carbonitrile

(2) 5-(4-{[4-Amino-5-cyano-7-(3,4-dihydroxy-5-hydroxymethyl-3-methyl-tetrahydrofuran-2-yl)-7H-pyrrolo[2,3-d]pyrimidin-6-ylamino]-methyl}-biphenyl-2-yloxy)-pent anoic acid ethyl ester

(3) 4-Amino-6-{[2-(4-chloro-butoxy)-biphenyl-4-ylmethyl]-amino}-7-(3,4-dihydroxy-5-hydroxymethyl-3-methyl-tetrahydro-furan-2-yl)-7H-pyrrolo[2,3-d]pyrimidine-5-carbonitrile

(4) 4-Amino-7-(3,4-dihydroxy-5-hydroxymethyl-3-methyl-tetrahydro-furan-2-yl)-6-{[2-(4-morpholin-4-yl-butoxy)-biphenyl-4-ylmethyl]-amino} -7H-pyrrolo[2,3-d]pyrimidine-5-carbonitrile

( 5 ) 4-Amino-6-({2-[4-(tert-butyl-dimethyl-silanyloxy)-butoxy]-biphenyl-4-ylmethyl}-amino)-7-(3,4-dihydroxy-5-hydroxymethyl-3-methyl-tetrahydro-furan-2-yl)-7H-pyrrolo[2,3-d]pyrimidine-5-carbonitrile

(6) 4-Amino-7-(3,4-dihydroxy-5-hydroxymethyl-3-methyl-tetrahydro-furan-2-yl)-6-{[2-(4-piperidin-1-yl-butoxy)-biphenyl-4-ylmethyl]-amino}-7H-pyrrolo [2,3-d]pyrimidinc-5-carbonitrilc

( 7 ) 5-(4-{[4-Amino-5-cyano-7-(3,4-dihydroxy-5-hydroxymethyl-3-methyltetrahydrofuran-2-yl)-7H-pyrrolo[2,3-d]pyrimidin-6-ylamino]-methyl}-biphcnyl-2-yloxy)-pentanoic acid dimethylamide

(8) 4-Amino-7-(3,4-dihydroxy-5-hydroxymethyl-3-methyl-tetrahydro-furan-2-yl)-6-{[2-(4-hydroxy-butoxy)-biphenyl-4-ylmethyl]-amino}-7H-pyrrolo[2,3-d]pyrimidine-5-carbonitrile

(9) 5-(4-{[5-Cyano-7-(3,4-dihydroxy-5-hydroxymethyl-3-methyltetrahydro-furan-2 yl)-4-hydroxy-7H-pyrrolo[2,3-d]pyrimidin-6-ylamino]methyl}-biphenyl-2-yloxy)-pentanoic acid ethyl ester

The compound as the above-mentioned (1) and (2) - - - (9) is quoted as follows as compound (1) compound (2) - - - compound (9), respectively.

A general synthesis method of the compound represented by general formula (I) in the present invention is explained. This inventive compound is prepared by the following methods. The protective group used for manufacturing include the ones described in WILEY-Interscience published "Protective Groups in Organic Synthesis (1999)".

The outline of the synthesis method of the compounds represented by general formula (I)(wherein, L is N-$R^1$ and $R^1$ is hydrogen atom) is shown below.

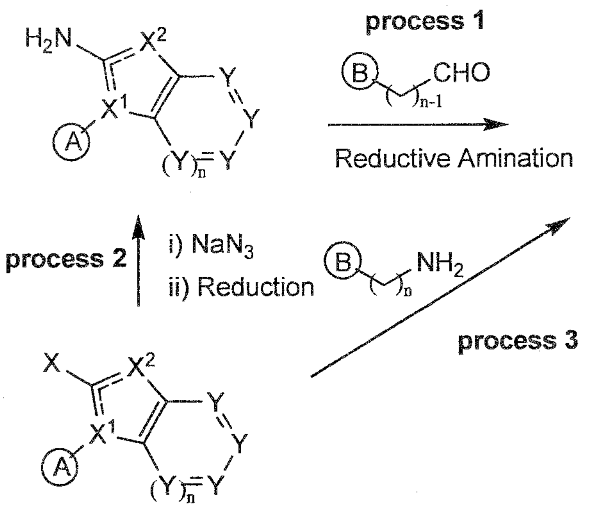


A manufacturing process 1 to 7 of the compound described in the above is explained as follows.

(1) Process 1

**process 1**

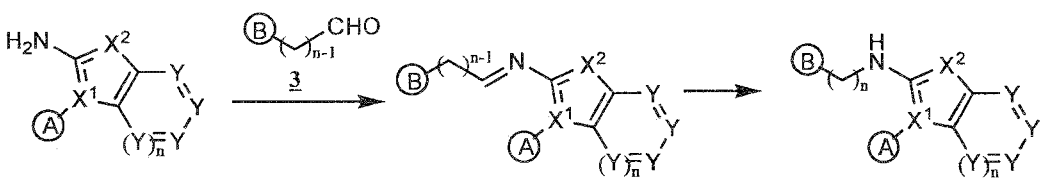

2 4 1

An imine represented by the above general formula 4 can be prepared by subjecting a compound represented by the above general formula 2 to condensation with a compound represented by the above general formula 3 under without solvent or in an inert solvent. As the inert solvent used in the reaction, for example toluene, benzene, xylene, N,N-dimethylformamide, tetrahydrofuran, dimethylsulfoxide, chloroform, dichloromethane, a mixed solvent thereof or the like can be illustrated. The reaction temperature is usually from room temperature to reflux temperature, if necessary the water generated by the reaction is removed off using a Dean-Stark. The reaction time is usually from several times to few days, varying based on a used starting material, solvent and reaction temperature.

Compounds represented by the above general formula 1 can be prepared by subjecting a compound represented by the above general formula 4 according to a general reduction method of a imine to reduction using a reducing agent such as sodium borohydride, potassium borohydride, sodium cyanoborohydride or sodium triacetoxyborohydride in an inert solvent. As the inert solvent used in the reaction, for example tetrahydrofuran, N,N-dimethylformamide, dimethylsulfoxide, chloroform, dichloromethane, toluene, a mixed solvent thereof or the like can be illustrated. The reaction temperature is usually from room temperature to reflux temperature, preferably room temperature. The reaction time is usually from 30 minutes to few days, varying based on a used starting material, solvent and reaction temperature.

In this process, the imine may be reduced after isolation, but it also reduced without isolation.

The compounds represented by the above general formula 2 which is used as a starting material in the present production process are commercially available or can be prepared in a known or similar method (for examples, the methods described in J. Org. Chem., 1981, 46(13) 2891-2823., Chem. Pharm. Bull., 1977, 25(4), 575-578., Tetrahedron, 1970, 26, 4251-4259. etc.).

A manufacturing example is explained in the following process 2.

(2) Process 2

**process2**

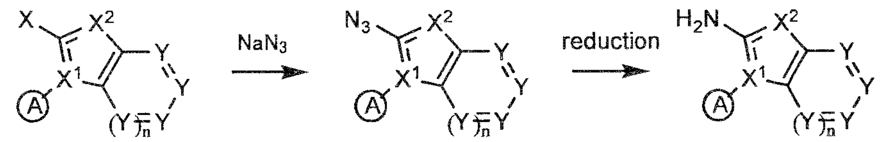


5 6 2

A compound represented by the above general formula 5(wherein, X is halogen) is reacted with sodium azide in an inert solvent to yield a compound represented by the above general formula 6. As the inert solvent used in the reaction, for example, diethyl ether, tetrahydrofuran, N,N-dimethylformamide, dimethylsulfoxide, chloroform, dichloromethane, toluene, a mixed solvent thereof or the like can be illustrated, preferably N,N-dimethylformamide. The reaction temperature is usually from 0°C to reflux temperature, preferably 150°C. The reaction time is usually from 1hour to few days, varying based on a used starting material, solvent and reaction temperature.

A compound represented by the above general formula 6 is reduced according to a general reduction method of an azide to reduction using a metal catalyst such as palladium on carbon, platinum oxide or the like in the presence or absence of an acid such as hydrochloric acid or the like in an inert solvent. As the inert solvent used in the reaction, for example diethyl ether, tetrahydrofuran, N,N-dimethylformamide, dimethylsulfoxide, chloroform, dichloromethane, toluene, alcohol such as ethanol, a mixed solvent thereof or the like can be illustrated, prefer ethanol. The reaction

temperature is usually from 0°C to reflux temperature, preferably room temperature. The reaction time is usually from one hour to few days, varying based on a used starting material, solvent and reaction temperature.

(3) process3

process3

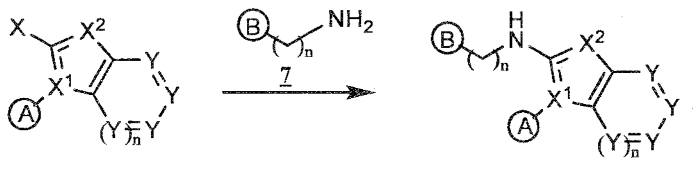


5 1

A compound represented by the above general formula 1 can be also prepared by subjecting a compound represented by above general formula 5 to condensation with a compound represented by above general formula 7 in the presence of base such as triethylamine, N,N-diisopropylethylamine, potassium carbonate, sodium carbonate, sodium hydroxide, sodium methoxide or the like in an inert solvent. As the inert solvent used in the reaction, for example, tetrahydrofuran, N,N-dimethylformamide, dimethylsulfoxide, chloroform, dichloromethane, toluene, alcohol such as 2-propanol, a mixed solvent thereof or the like can be illustrated, preferably 2-propanol. The reaction temperature is usually from 0°C to reflux temperature, preferably 100°C. The reaction time is usually from one hour to few days, varying based on a used starting material, solvent and reaction temperature.

(4) process4

process4

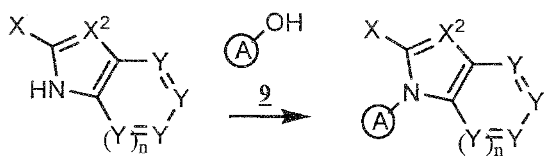


8 5

9 :

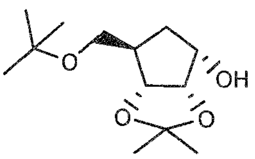

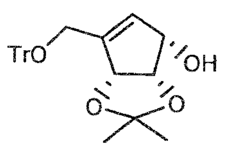

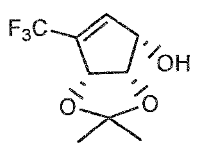

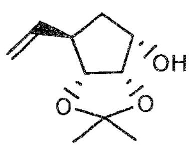

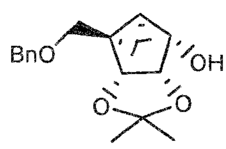

A compound represented by the above general formula 5 can be prepared by subjecting a compound represented by above general formula 8 to condensation with sugar derivatives represented by above general formula 9 (for example, the methods described in Tetrahedron Lett., 1999, 40, 3309-3312.,J. Org. Chem., 1999, 64, 4172-4178., Bioorg. Med. Chem. Lett., 2006, 16, 285-287., J. Med. Chem., 2001, 44, 3985-3993., Tetrahedron Lett., 1997, 38, 4207-4210., Tetrahedron Lett., 2005, 46, 8913-8915., Tetrahedron Lett., 2004, 8981-8982., J. Org. Chem., 2004, 69, 3993-3996., J. Med. Chem., 2006, 49, 1140-1148., Org. Lett., 2001, 3, 597-599., NUCLEOSIDE&NUCLEOTIDE, 1994, 13, 2035-2050.) according to general Mitsunobu condition in an inert solvent.

The reaction is carried out by using a Mitsunobu reagent such as diethyl azodicarboxylate, diisopropyl azodicarboxylate, dicyclohexyl azodicarboxylate or the like in the presence of a phosphine reagent such as triphenylphosphine,

tributyllphosphine or the like. As the inert solvent used in the reaction, for example, tetrahydrofuran, N.N-dimethylformamide, dimethylsulfoxide, chloroform, dichloromethane, toluene, a mixed solvent thereof or the like can be illustrated. The reaction temperature is usually from 0°C to reflux temperature, preferably room temperature. The reaction time is usually from thirty minutes to few days, varying based on a used starting material, solvent and reaction temperature.

Moreover even if phosphorane such as cyanomethylenetributylphosphorane, cyanomethylenetrimethylphosphoran or the like is used, a similar reaction can be done without the phosphine reagent. As the solvent used in the reaction, for example, benzene, toluene, a mixed solvent thereof or the like can be illustrated. The reaction temperature is usually from room temperature to reflux temperature. The reaction time is usually from thirty minutes to few days, varying based on a used starting material, solvent and reaction temperature.

(5) process5

process5

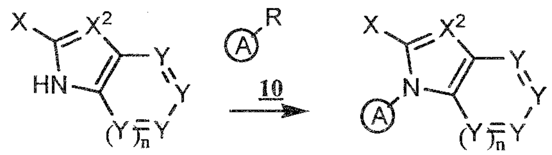


8 5

10 :

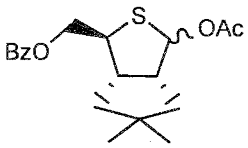

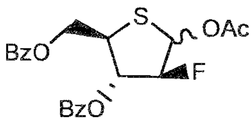

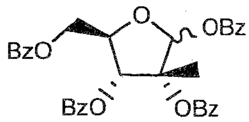

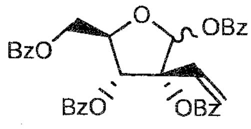

A compound represented by the above general formula 5 can also be prepared by subjecting a compound represented by above general formula 8 to coupled with sugar derivatives represented by above general formula 10, wherein, R represents the leaving group(for example, a compound obtained by the methods described in J. Org. Chem., 1997, 62, 1754-1759., J. Org. Chem., 2006, 71 4018-4020., J. Med. Chem., 2006, 49, 273-281., J. Org. Chem., 2006, 71, 921-925., WO 2004/002422., WO 03/068162.). A compound represented by the above general formula 8 is pretreated with a silylated reagent such as N,O-Bis(trimethylsilyl)acetamide, N,N'-Bis(trimethylsilyl)urea, trimethylsilyl chloride, hexamethyldisilazane or the like or a base such as 1,8-Diazabicyclo[5.4.0]undee-7-ene or the like, followed by coupling reaction in the presence of a Lewis acid such as Trimethylsilyl Trifluoromethanesulfonate, tin(IV) chloride or the like in an inert solvent. As the inert solvent used in the reaction, for example, acetonitrile, tetrahydrofuran, dichloromethane, N,N-dimethylacetamide, N,N-dimethylformamide, dimethylsulfoxide, a mixed solvent thereof or the like can be illustrated, desirability is acetonitrile. The reaction temperature is usually from 0°C to reflux temperature, desirability is room temperature. The reaction time is usually from one hour to few days, varying based on a used starting material, solvent and reaction temperature.

A compound represented by the above general formula 3 is commercially available or can be prepared in a known or similar method.A manufacturing example is explained in the following process 6.

(6) process6

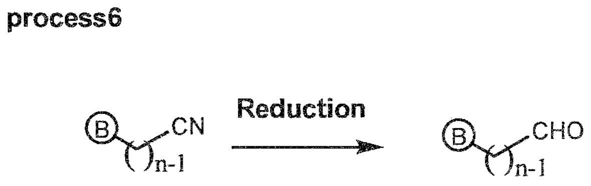

11 3

A compound represented by the above general formula 3 can be prepared by subjecting a compound represented by the above general formula 11 according to a general reduction method of a nitrile to reduction using a reducing agent such as diisobutylaluminium hydride or the like in an inert solvent followed by saponification using a acid such as hydrochloric acid or the like. As the inert solvent used in the reaction, for example diethyl ether, tetrahydrofuran, dichloromethane, toluene, hexane, a mixed solvent thereof or the like can be illustrated, prefer dichloromethane. The reaction temperature is usually from 0°C to reflux temperature, preferably room temperature. The reaction time is usually from 1 hour to few days, varying based on a used starting material, solvent and reaction temperature.

( 7 ) process7

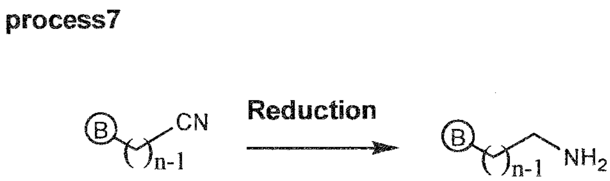

11 7

A compound represented by the above general formula 7 can be prepared by subjecting a compound represented by the above general formula 11 according to a general reduction method of a nitrile to reduction using a reducing agent such as lithium aluminium hydride, sodium borohydride, lithium borohydride, or the like in an inert solvent. As the inert solvent used in the reaction, for example diethyl ether, tetrahydrofuran, dichloromethane, toluene, methanol, ethanol, a mixed solvent thereof or the like can be illustrated. The reaction temperature is usually from 0°C to reflux temperature, prefer room temperature. The reaction time is usually from 1 hour to few days , varying based on a used starting material, solvent and reaction temperature.

This invention is concerning a pharmaceutical composition comprising as an active ingredient a compound represented by the above general formula (I), or a pharmaceutically acceptable salt thereof, or a prodrug thereof, or a hydrate or solvate thereof. This pharmaceutical composition can be used in combination with at least one agent for the prevention or treatment of a disease associated with an abnormality of plasma uric acid level. Examples of such agents include a colchicine for the prevention or treatment of gout, a nonsteroidal anti-inflammatory agent such as indomethacin, naproxen, fenbufen, pranoprofen, oxaprozin, ketoprofen, etoricoxib, tenoxicam or the like, a uric acid synthesis inhibitor such as allopurinol, oxypurinol, febuxostat, FYX-051, Y-700 or the like, a uricosuric drug such as probenecid, bucolome, benzbromarone or the like, a urinary alkalinizer such as sodium hydrogen carbonate, potassium citrate, sodium citrate or the like, a diuretic such as chlorothiazide, trichloromethiazide, furosemide, triamterene or the like, an AT-1 receptor antagonist such as losartan, irbesartan or the like, a calcium channel blocker such as diltiazem, verapamil, amlodipine or the like, an ACE inhibitor such as captopril, cirazapril or the like, a biguanide such as metformin, phenformin or the like, a PPAR ligand agonist or antagonist such as rosiglitazone, troglitazone, pioglitazone, ONO-5129, KL-111, MCC-555 or the like, an α-glucosidase inhibitor such as miglitol, acarbose, voglibose or the like, a DPP-IV inhibitor such as sitagliptin, TS-021, SYR-322, TA-6666, vildagliptin or the like, a insulinotropic agent such as chlorpromazine, glimepiride, tolbutamide or the like, a lipase inhibitor such as orlistat or cetilistat, β-3 adrenoceptor agonist such as CL316243, cannabinoid antagonist such as rimonabant, an All receptor antagonist such as losartan, candesartan, telmisartan or the like or an antiobestic drug such as sibutramine, S - 2367 or the like.

The tablet, the powder, the granule, or the like are used as a solid constituent for the oral administration of this inventive compound. In such a solid composition, one or any more an activator is mixed with at least one inert diluent, for example lactose, mannitol, glucose, hydroxypropylcellulose, microcrystalline cellulose, soluble starch, povidone,

magnesium aluminometasilicate. The excipients, for example, the lubricant such as magnesium stearate, the disintegrator such as calcium carboxymethylcellulose, the stabilizer such as lactose or the solubilizer such as glutamic acid or aspartic acid, other than an inert diluent may be contained in this composition according to the common procedure. The tablet or the pill may be covered with the sugar coating such as saccharose, gelatin, hydroxypropylcellulose, hydromellose phthalate, or the like , the gastric and the enteric film if necessary.

A liquid composition for the oral administration contains a pharmaceutically acceptable emulsion, solution, suspension, syrup, elixir or the like, and contains the generally used inert diluent, for example, a purified water and ethyl alcohol. This composition may be contained the adjuvant, the sweetening agent, the flavor, the aromatic agent and the preservative such as the solubilizer, the solubilization agent, the moistening agent and suspension besides an inert diluent.

A injection for the non-oral administration contains the sterilized water soluble or water insoluble solution, suspension and emulsion. As a diluent of water soluble solution and suspension, for example, a distilled water and a water for injection are contained. As a diluent of water insoluble solution and suspension, for example, a vegetable oil such as propylene glycol, polyethylene glycol, olive oil, an alcohols such as ethylalcohol, a polysorbate 80 or the like are contained.

In addition, these compositions may be contained the excipient such as the tonicity adjusting agent, the preservative, the moistening agent, the emulsifier, the dispersant, the stabilizer(for example, lactose), the solubilization agent or solubilizer. For example, these are sterilized by the filtration that passes the bacteria-retaining filter, the mixing of a germicide or the UV irradiation. After the sterilized solid composition prepared, these can be also used by dissolving to sterile water or sterilized water of injection before it uses.

Test Examples and Examples of the compound represented by general formula (I) in the present invention is explained as follows.

Test Example 1

Measurement of Inhibitory Activity against Uptake of Adenosine Through Human CNT2

An Uptake buffer was prepared by addition of a mixture of non-radioisotope labeled (Sigma) and $^{14}C$-labeled (GE Healthcare) adenosine at the final concentration of 10 μM into a buffer, pH 7.4, containing 140 mM sodium chloride, 2 mM potassium chloride, 1 mM calcium chloride, 1 mM magnesium chloride, 10 mM HEPES 2-[4-(2-hydroxyethyl)-1-piperazinyl]ethane sulfonic acid, 5 mM tris(hydroxymethyl)aminomethane and 5 mM glucose. For measurement of basal uptake, Basal uptake measurement buffer, which contained 140 mM choline chloride instead of sodium chloride was prepared. In uptake assays, NBMPR was added to Uptake buffer and Basal uptake measurement buffer at the final concentration of 10 μM. In the case of measurement of inhibitory activity of test compounds, test compounds were dissolved in dimethyl sulfoxide, and then appropriately diluted with Uptake buffer as to Measurement buffers. After removing culture medium of human CNT2 transiently expressing cells, Pretreatment buffer (Basal uptake measurement buffer without adenosine and glucose) was added to wells at 500 μL/well and incubated at 37 °C for 10 minutes. After repeating the same step again, Pretreatment buffer was removed and Measurement buffers and Basal uptake measurement buffer were added at 300 μL/well and incubated at 37 °C. After incubation for 30 minutes, Measurement buffers and Basal uptake measurement buffer were removed, and the cells were washed with Washing buffer (Basal uptake measurement buffer with non-radioisotope labeled adenosine at 10 μM) at 500 μL/well twice. The cells were solubilized with 0.5 mol/L sodium hydroxide at 200 μL/well, and the cell lysates were transferred into glass vials at 100 μL. After mixing with 5 mL of ACS- II (GE Healthcare Bioscience), radioactivity was measured by means of scintillation counter (Aloka). One hundred % was set to the difference between the uptake in the control group and the basal uptake, and the uptake of adenosine at each test compound concentration was calculated. The test compound concentration inhibiting 50 % uptake of adenosine ($IC_{50}$ value) was calculated using regression line. The results are shown in Table 1. The compounds (1)-(9) which have residue of non-sugar possessed high CNT2 inhibitory activity. The $IC_{50}$ value was expressed as A; 1~10 nM, B 10~100 nM or not less than 100 nM;

Table 1

| Compound No. | $IC_{50}$ value |
|---|---|
| Compound (1) | C |
| Compound (2) | B |
| Compound (3) | B |
| Compound (4) | B |
| Compound (5) | C |
| Compound (6) | B |

(continued)

| Compound No. | $IC_{50}$ value |
|---|---|
| Compound (7) | A |
| Compound (8) | A |
| Compound (9) | C |

Test Example 2

Four weeks old male SD rats (Charles river japan corporation) were purchased and the animals over 1 weeks quarantine were used. Body weight of the rats fasted 24 hours from last day were measured and grouped used grouping soft (Stat light #11, Yukums corporation). Test compound and [$^3$H]-adenosine solution (4 μCi/mL) were given orally at the volume of 2 mL/kg, and about 500 μL of blood were obtained from retro orbital vein. The blood was centrifuged at 3000 rpm for 10min, and 13 μL of $H_2O_2$ solution were added to the 100 μL of resulted serum and placed for 10 min. And 5 mL of liquid scintilation cocktail added and mixed enough, and radio activity was measured using liquid scintillation counter. Inhibitory rate was calculated according to the formula described as follow, and the regression line was obtained from inhibitory rate and its log dose. The 50 % inhibitory dose of the compound ($ED_{50}$ ) at the inhibition on absorption of adenosine was calculated used regression line formula The results were shown in Table 2. Compounds (1), (2), (3), (4), (7) and (8) which have residue of non-natural sugar showed high $ED_{50}$ value.

Inhibitory ratio = {1 - radio activity of the serum obtained from rats treated with test compound (dpm)/ radio activity of the serum obtained from rats treated with vehicle (dpm)} ×100

Table2

| Compound No. | $ED_{50}$ value |
|---|---|
| Compound (1) | 1.3 |
| Compound (2) | 1.4 |
| Compound (3) | 1.1 |
| Compound (4) | 0.51 |
| Compound (7) | 3.0 |
| Compound (8) | 0.78 |

Test Example 3

Stability of Nucleic acid transporter inhibitor in solution

0.02 mL of comparative compound or compound (1) methanol solution at the concentration of $10^{-3}$ M was added to the 1.98 mL of acetate buffer, pH 3 (final concentration of $10^{-5}$ M). The solutions were incubated at 25 □, and the reaction solutions were collected at 2, 4 and 20 hours later. Each obtained 0.5 mL of reaction solutions were neutralized and adjusted to 1 mL, and 0.01 mL of $10^{-3}$ M methanol solutions of 4-hydroxy were spiked as internal standard substrate. The solutions were measured by HPLC according to the measurement condition shown as follows, and the ratio ( IS ratio ) compared the amount of internal standard substrate with the amount of compounds was calculated. The remaining rate of test compounds were obtained to compare the IS ratio with IS ratio of pre-incubation according to the method described above. The remaining rate of comparative compounds and compound (1) at 20 hours after were shown in Table 3. And the changes of remaining rate of comparative compounds and compound (1) at 2, 4 and 20 hours after were shown in Figure 1. Stability of Compound (1) which has residue of non-natural sugar in pH3 solution is better than that of comparative compound which has residue of natural sugar.

HPLC Analytical Condition

- column : Inertsil ODS-2 (φ4.6×150 mm)
- Mobile phase 20 mM phosphate buffer (pH 6) /CH3CN = 7/3 (w/w)
- Detection absorbance : 250 nm
- Flow rate : 1 mL/min
- Injection volume : 0.02 mL
- Time span of measurement : 20 min

Table3

| pH of solution | residual ratio of the compound (%) | |
|---|---|---|
| | compound(1) | Comparative compound |
| 3 | 85.0 | 45.0 |

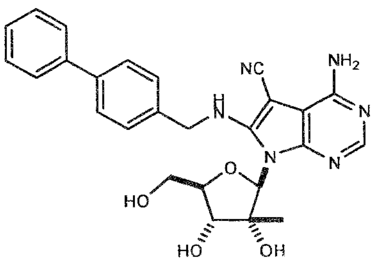

compound(1)

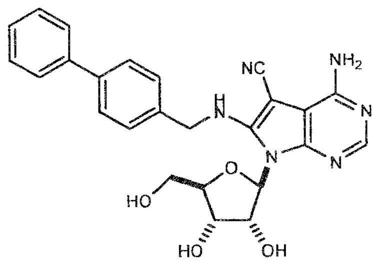

Comparative Compound

The present invention is further illustrated in more detail by way of the following Reference Examples and Examples. The Reference Examples is an example of having shown the manufacturing process of the starting material used in the Examples.

Reference Example 1

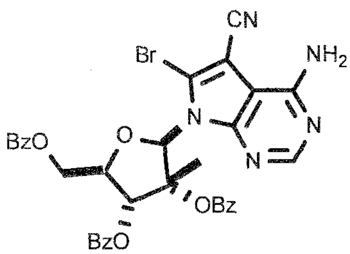

To a mixture of 1,2,3,5-tetra-O-benzoyl-2-C-methyl-D-ribofuranose (10.282 g), 4-amino-6-bromo-7H-pyrro[2,3-d]pyrimidine-5-carbonitrile (4.215 g) and acetonitrile (180 mL) was added DBU (8.0 mL) and TMSOTf (12.8 mL) at 0°C, and resulting mixture was stirred at 60°C. for 17 hours. The reaction mixture was cooled to room temperature and poured into saturated aqueous sodium hydrogen carbonate. The mixture was extracted with ethyl acetate. The organic layer was washed brine, dried over anhydrous sodium sulfate and evaporated. The residue was purified by silica gel column

chromatography (ethyl acetate/chloroform) to give the title compound as a pale yellow solid (5.394 g). $^1$H-NMR($CDCl_3$)δ= 1.68(s, 3H), 4.78-4.90(m, 3H), 5.66(br-s, 2H), 6.87(s, 1H), 7.26-7.33(m, 1H), 7.44-7.52(m, 6H), 7.59-7.64(m, 3H), 7.94-7.97(m, 2H), 8.12-8.15(m, 4H),8.39(s, 1H).

Reference Example 2

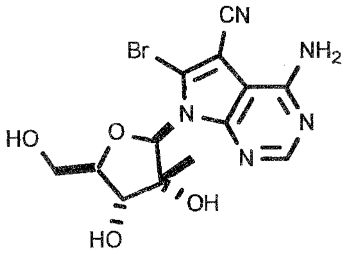

To a solution of the compound (Reference Example 1) (4.394 g) in methanol (130 mL) was added sodium methoxide (171 mg) at room temperature, and resulting mixture was stirred for 4.5 hours. The reaction mixture was added activating Dowex to pH=5 then it was filtered to remove precipitates. The filtrate was concentrated under reduced pressure. The residue was purified by silica gel column chromatography (chloroform/ methanol) to give the title compound as a white solid (1.036 g). $^1$H-NMR(DMSO-$d_6$)δ= 0.82(s, 3H), 3.75-3.90(m, 3H), 4.40(br-s, 1H), 4.93(m, 1H), 5.17(m, 1H), 5.30-5.32(m, 1H), 6.03(br-s, 1H), 7.04(br-s, 2H), 8.17(s, 1H).

Example 1

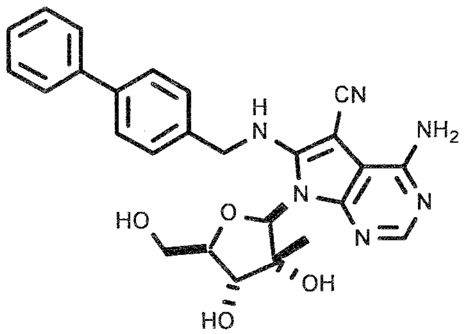

The compound (1) represented by the above formula was synthesized by the following method.

To a solution of the compound (Reference Example 2) (200 mg) in isobutanol (5 mL) was added C-biphenyl-4-yl-methylamine (229 mg) and N,N'-diisopropylethylamine (0.28 mL). The resulting mixture was refluxed for 14 hours. The solvent was removed under reduced pressure. The residue was purified by silica gel column chromatography (chloroform/ methanol) to give the title compound (1) as a pale yellow solid (63 mg).
$^1$H-NMR(DMSO-$d_6$)δ= 0.72(s, 3H), 3.71-3.74(m, 1H), 3.86-3.90(m, 2H), 4.03-4.05(m, 1H), 4.64-4.73(m, 2H), 5.07(s, 1H), 5.37(d, J=6.8Hz,1H), 5.94(m, 1H), 6.18(br-s, 2H), 6.40(br-s, 1H), 7.33-7.37(m, 1H), 7.43-7.47(m, 4H), 7.66(d, J=8.1Hz, 4H), 8.03(s, 1H), 8.14(m, 1H).

The compounds (2) to (6) were prepared in a similar manner to that described in Example 1 using the corresponding materials.

Compound (2)

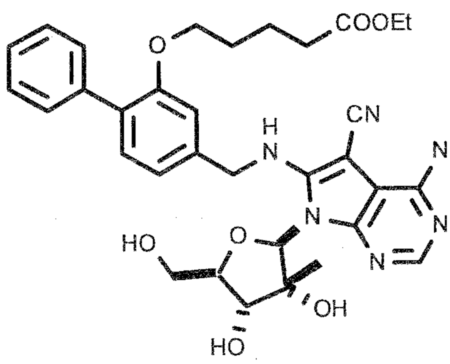

$^1H$-NMR($CD_{3O}D$)δ= 0.86(S, 3H), 1.19(t, J=7.1Hz, 3H), 1.67(m, 4H), 2.23(t, J=7.1Hz, 2H), 3.88-4.08(m, 7H), 4.19(br-s, 1H), 4.63-4.80(m, 2H), 6.51(br-s, 1H), 7.08-7.46(m, 8H), 8.03(s, 1H).

Compound (3)

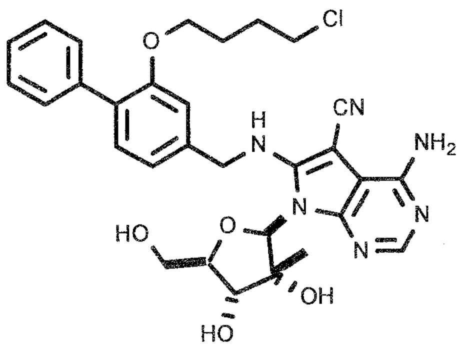

$^1H$-NMR(CD3OD)δ= 0.86(S, 3H), 1.80-1.83(m, 4H), 3.47(t, J=6.3Hz, 2H), 3.87-4.21(m, 6H), 4.64-4.80(m, 2H), 6.51 (br-s, 1H), 7.10(d, J=7.8Hz, 1H), 7.17(s, 1H), 7.25-7.28(m, 2H), 7.34(t, J=7.3Hz, 2H), 7.45-7.47(m, 2H), 8.03(s, 1H).

Compound (4)

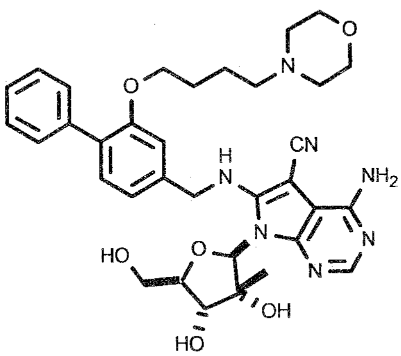

$^1H$-NMR($CD_3OD$)δ= 0.86(S, 3H), 1.55-1.62(m, 2H), 1.68- 1.75(m, 2H), 2.36-2.43(m, 6H), 3.64-3.66(m, 4H), 3.87- 4.21 (m, 6H), 4.65-4.80(m, 2H), 6.51 (br-s, 1H), 7.09(dd, J=1.5Hz, 7.8Hz, 1H), 7.17(s, 1H), 7.24-7.27(m, 2H), 7.34(t, J==7.1Hz, 2H), 7.47(dd, J=1.5Hz, 8.5Hz, 2H), 8.03(s, 1H).

Compound (5)

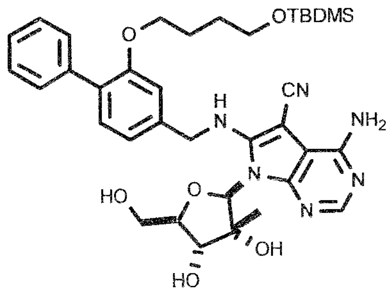

$^1$H-NMR($CD_3OD$)δ= 0.01(s, 6H), 0.86-0.91(m, 11H), 1.56- 1.76(m, 4H), 3.58(t, J=5.9Hz, 2H), 3.88-4.22(m, 6H), 4.66-4.82(m, 2H), 6.52(br-s, 1H), 7.08-7.11(m, 1H), 7.17(s, 1H), 7.24-7.28(m, 2H), 7.32-7.36(m, 2H), 7.47-7.49(m, 2H), 8.04 (s, 1H).

Compound (6)

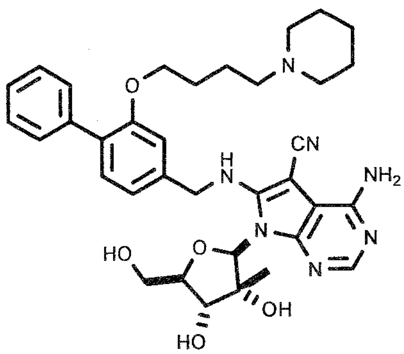

$^1$H-NMR($CD_3OD$)δ= 0.86(s, 3H), 1.29-1.68(m, 10H), 2.21-2.34(m, 6H), 3.87- 4.21(m, 6H), 4.65-4.76(m, 2H), 6.51(s, 1H), 7.09(d, J=7.6Hz, 1H), 7.17(s, 1H), 7.26(d, J=7.8Hz, 2H), 7.34(t, J=7.3Hz, 2H), 7.47(d, J=7.1Hz, 2H), 8.03 (s, 1H).

Example 2

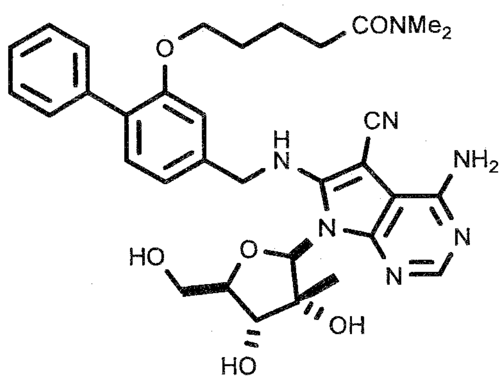

To a solution of the compound (2) (50 mg) in ethanol (2 mL) was added 10% sodium hydroxide (0.2 mL) at room temperature, and resulting mixture was stirred for 1 hours. The reaction mixture was added activating Dowex to pH=5 then it was filtered to remove precipitates. The filtrate was concentrated under reduced pressure. To a solution of the in N,N-dimethylformamide was added dimethylamine hydrochloride (34 mg), 1-hydroxy-1H-benzotriazole monohydrate (38 mg), 1-ethyl-3-(3-dimethylamino-propyl)carbodiimide hydrochloride (48 mg) and triethylamine (0.12 mL) at room temperature, and resulting mixture was was stirred for 13.5 hours. The reaction mixture was poured into water. The mixture was extracted with ethyl acetate. The organic layer was washed brine, dried over anhydrous sodium sulfate and evaporated. The residue was purified by silica gel column chromatography (chloroform/methanol) to give the title compound (7) as a pale yellow solid (36 mg).
$^1$H-NMR($CD_3OD$)δ= 0.86(s, 3H), 1.61-1.76(m, 4H), 2.29(t, J=7.6Hz,2H), 2.86-2.88(m, 6H), 3.87- 4.21(m, 6H), 4.67(d, J=16.1Hz,1H), 4.88(d, J=16.1Hz,1H), 6.511(br-s, 1H), 7.10(dd, J=1.5Hz, 7.8Hz, 1H), 7.17(s, 1H), 7.23-7.27(m, 2H), 7.32-7.35(m, 2H), 7.47-7.49(m, 2H), 8.03(s, 1H).

Example 3

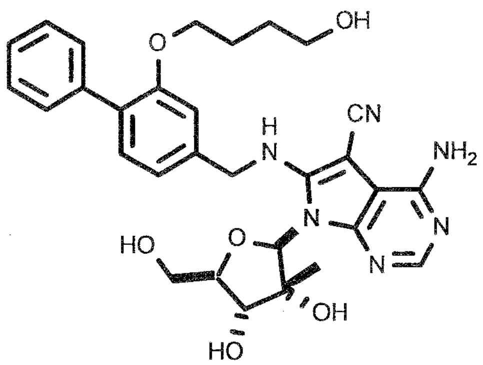

To a solution of the compound (5) (233 mg) in methanol (3 mL) was added ammonium fluoride(376 mg) at room temperature, and resulting mixture was stirred at 60°C. for 16 hours. The reaction mixture was concentrated under reduced pressure. The residue was purified by silica gel column chromatography (chloroform/methanol) to give the title compound (8) as a yellow solid (130 mg).
$^1$H-NMR($CD_3OD$)δ= 0.86(s, 3H), 1.55-1.62(m, 2H), 1.71-1.78(m, 2H), 3.50(t, J=6.3Hz,2H), 3.87-4.21(m, 6H), 4.67(d, J=15.9Hz,1H), 4.78(m,1H), 6.51(br-s, 1H), 7.09(dd, J=1.7Hz, 7.8Hz, 1H), 7.17(s, 1H), 7.23-7.27(m, 2H), 7.33(t, J=7.3Hz, 2H), 7.47 (dd, J=1.5Hz, 8.5Hz, 2H), 8.03(s, 1H).

Example 4

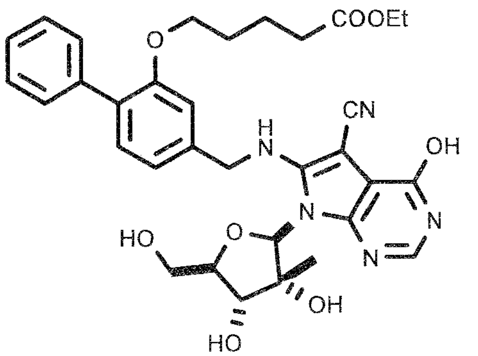

To a solution of the compound (2) (1.165g) in acetic acid (30 mL) was added a solution of sodium nitrite (376

mg) in water (10 mL) at room temperature, and resulting mixture was stirred for 12 hours. The reaction mixture was concentrated under reduced pressure. The residue was purified by silica gel column chromatography (chloroform/methanol) to give the title compound (9) as a yellow solid (525 mg). $^1$H-NMR(DMSO-$d_6$)δ= 0.76(s, 3H), 1.15(t, J=7.1Hz, 3H), 1.59-1.67(m, 4H), 2.26(t, J=7.3Hz, 2H), 3.71- 4.11(m, 8H), 4.55-4.68(m, 2H), 5.08(s, 1H), 5.37(d, J=7.1Hz, 1H), 5.94 (br-s, 1H), 6.32(s, 1H), 7.03(d, J=7.6Hz, 1H), 7.13(s, 1H), 7.25-7.39(m, 4H), 7.48(d, J=7.3Hz, 2H), 7.88(m, 2H), 12.24 (br-s, 1H).

Industrial Applicability

The compounds represented by the general formula (I) in the present invention possess the excellent CNT2 inhibitory activity and suppress the systemic absorption of the purinenucleoside. Therefore, they are useful as agents for the decreased of plasma uric acid level.

## Claims

**1.** The compounds have the following general formula (I);

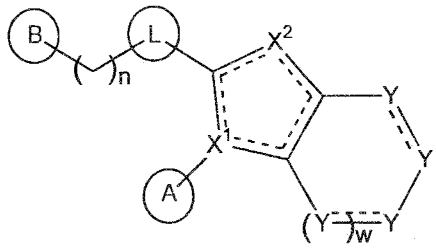

——— ( I )

wherein,
n and w represent 0 to 5,
--- represents a single bond or a double bond,
$X^1$ represents N or C, provided that in the case where $X^1$ is N, $X^2$ represents C-R and in the case where $X^1$ is C, $X^2$ represents N-$R^1$, $CR^1R^2$, O or S,
wherein, R represents hydrogen atom, lower alkyl group having one to five carbon atoms, lower alkyl group including cycloalkyl ring having three to five carbon atoms, lower alkenyl group having two to five carbon atoms, lower alkynyl group having two to five carbon atoms, lower haloalkyl group having one to five carbon atoms, lower alkoxyalkyl group having two to five carbon atoms, lower hydroxyalkyl group having one to five carbon atoms, lower alkoxycarbonyl group having two to five carbon atoms, lower aminoalkyl group having one to five carbon atoms, hydroxy group, amino group, mercapto group, carboxyl group, carbamoyl group, alkanoyl group, cyano group or halogen group, $R^1$ and $R^2$ may be either the same or different and represent hydrogen atom, lower alkyl group having one to five carbon atoms or lower haloalkyl group having two to five carbon atoms,
L represents N-$R^1$, $CR^1R^2$, lower alkenylene group having two to five carbon atoms, lower alkynylene group having two to five carbon atoms or $S(O)_v$, wherein, $R^1$ and $R^2$ are same meaning as defined in the above, v represents 0 to 2,
Y's may be either the same or different and represent $CH_2$, CH, O, S, N-$R^1$, CH-$NH_2$, CH-OH, CH-SH, CH-halogen atom, C-$NH_2$, C-OH, C-SH or C-halogen atom,
A represents the following general formula;

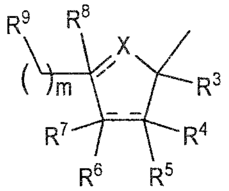

or

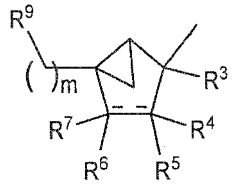

wherein,

m represents 0 to 1,
---- represents a single bond or a double bond,
X represents CH, $CH_2$, O, $S(O)_v$, N-$R^1$, $CF_2$ or -(=$CH_2$)-,
$R^3$, $R^4$, $R^5$, $R^6$, $R^7$, $R^8$ and $R^9$ may be either the same or different and represent hydrogen atom, hydroxyl group, lower alkylamino group having one to five carbon atoms, mercapto group , -$(CH_2)_u$-lower alkoxy group having one to five carbon atoms, halogen atom, lower haloalkyl group having one to five carbon atoms , lower alkyl group having one to five carbon atoms , lower alkyl group including cycloalkyl ring having three to five carbon atoms, lower alkenyl group having two to five carbon atoms or lower alkynyl group having two to five carbon atoms, $R^3$, $R^4$, $R^5$, $R^6$, $R^7$, $R^8$ and $R^9$ optionally formed cyclo or heterocyclo ring,
u represents 0 to 5,

however, in the general formula (I), in the case where $X^1$ is N, $X^2$ is C-CN, X is O, ---- is a single bond and $R^5$, $R^6$ and $R^9$ are hydroxyl group, $R^4$ is not hydrogen atom ,

B represents the following general formula;

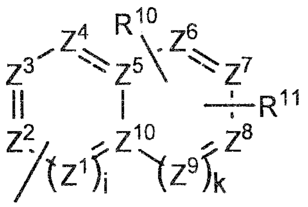

or

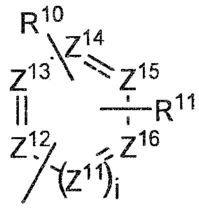

wherein,
i and k represent 0 to 5,
---- represents a single bond or a double bond,
$Z^1$, $Z^2$, $Z^3$, $Z^4$, $Z^5$, $Z^6$, $Z^7$, $Z^8$, $Z^9$, $Z^{10}$, $Z^{11}$, $Z^{12}$, $Z^{13}$, $Z^{14}$, $Z^{15}$ and $Z^{16}$ may be either the same or different and represent CH, $CH_2$, O, S, N or NH,
$R^{10}$ and $R^{11}$ may be either the same or different and represent hydrogen atom, halogen atom, lower alkyl group having one to five carbon atoms, optionally substituted cycloalkyl group having three to eight carbon atoms, optionally substituted heterocycloalkyl group, lower haloalkyl group having one to five carbon atoms, lower alkenyl group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, nitro group, cyano group, optionally substituted mono or bicyclic aromatic ring having five to ten carbon atoms, optionally substituted mono or bicyclic heteroaromatic ring having one to ten carbon atoms, optionally substituted benzyl group, optionally substituted amino group which can be optionally formed a quaternary ammonium compound, -CON$R^{12}R^{13}$, -NHCO$R^{15}$, -$(CH_2)_o$-$R^{15}$-$(CH_2)_p$-$R^{16}$, -lower alkenylene group having two to five carbon atoms-$R^{15}$, -lower alkynylene group having two to five carbon atoms-$R^{15}$, -$(CH_2)_o$-cycloalkyl group having three to eight carbon atoms-$(CH_2)_p$-$R^{15}$, -$(CH_2)_o$-heterocycloalkyl group-$(CH_2)_p$-$R^{15}$, -$(CH_2)_o$-NHC(=NH)-$NH_2$, -$(CH_2)_o$-C(=NH)-$NH_2$, -$(CH_2)_o$-D-$(CH_2)_t$-E-$(CH_2)_p$-$R^{15}$ or a group represented by the following general formula;

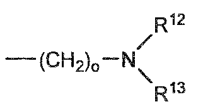 , 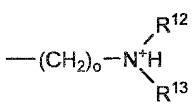 , 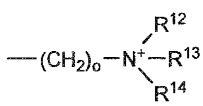 , 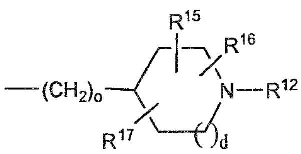 ,

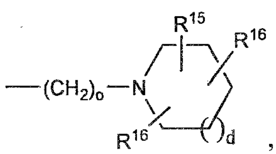 , 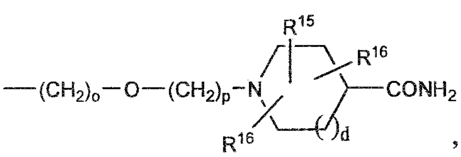 , 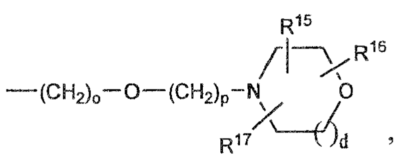 ,

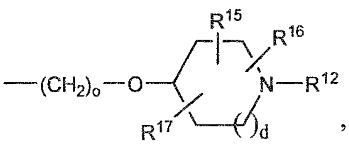 , 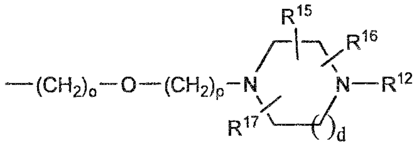 , 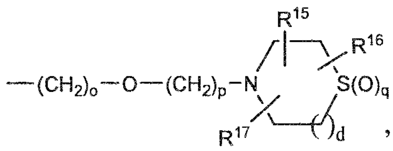 ,

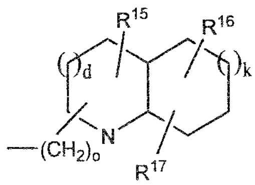 or 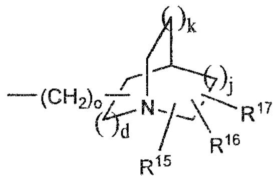

wherein,

o and p may be either the same or different and represent 0 to 15,

t represents 1 to 5,

q represents 0 to 2,

d, k and j represent 0 to 5,

D and E may be either the same or different and represent single bond(-), $-CONR^{12}-$, $-NR^{12}CO-$, -O-, $-NR^{12}-$, $-S(O)_q-$, $-NR^{12}S(O)_q-$, $-S(O)_qNR^{12}-$ or -NHC(=O)NH-,

$R^{12}$, $R^{13}$ and $R^{14}$ may be either the same or different and represent hydrogen atom, carboxyl group, lower alkyl group having one to five carbon atoms or lower hydroxy alkyl group having one to five carbon atoms and optionally formed the ring, $R^{15}$, $R^{16}$ and $R^{17}$ represent hydrogen atom, halogen atom, lower alkyl group having one to five carbon atoms, optionally substituted cycloalkyl group having three to eight carbon atoms, optionally substituted heterocycloalkyl group, lower haloalkynylene group having two to five carbon atoms, lower alkenylene group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, sulfonic acid group, phosphoric acid group, nitro group, cyano group, aminosulfonyl group, optionally substituted mono or bicyclic aromatic ring having five to ten carbon atoms, optionally substituted mono or bicyclic heteroaromatic ring having one to ten carbon atoms, optionally substituted benzyl group, $-S(O)_q$-lower alkyl group having one to five carbon atoms, $-CONR^{18}R^{19}$, optionally substituted amino group which can be optionally formed a quaternary ammonium compound, amino acid residue, sugar residue or a group represented by the following general formula;

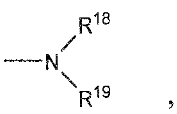 , 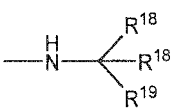 ,

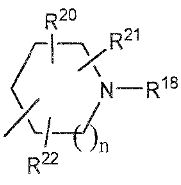 , 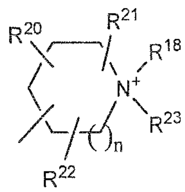 or 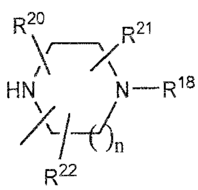

wherein,

$R^{18}$ and $R^{19}$ represent hydrogen atom, lower alkyl group having one to five carbon atoms, lower alkylcarbonyl group having two to five carbon atoms, lower alkylsulfonyl group having one to five carbon atoms, optionally substituted benzenesulfonyl group, lower alkoxycarbonyl group having two to five carbon atoms, lower hydroxyalkyl group having one to five carbon atoms, hydroxyethoxy group, alkoxyethoxy group, optionally substituted aminoethoxy group or $-(CH_2)_o-CONH_2$,

$R^{20}$, $R^{21}$ and $R^{22}$ represent hydrogen atom, halogen atom, lower alkyl group having one to five carbon atoms, optionally substituted cycloalkyl group having three to eight carbon atoms, optionally substituted heterocycloalkyl

group, lower haloalkynylene group having two to five carbon atoms, lower alkenylene group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, nitro group, cyano group, aminosulfonyl group, optionally substituted mono or bicyclic aromatic ring having five to ten carbon atoms, optionally substituted mono or bicyclic heteroaromatic ring having one to ten carbon atoms, optionally substituted benzyl group, $-S(O)_q$-lower alkyl group having one to five carbon atoms, optionally substituted amino group which can be optionally formed a quaternary ammonium compound,

$R^{23}$ represents lower alkyl group having one to five carbon atoms,

or a pharmaceutically acceptable salt thereof, or a prodrug thereof, or a hydrate or solvate thereof.

**2.** The compounds have the following general formula (II);

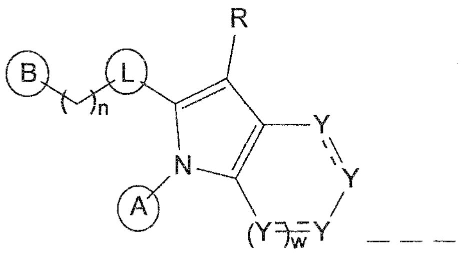
(II)

wherein,

A, B, L, R and Y are same meaning as defined in the above,

n and w represent 0 to 5,

---- represents a single bond or a double bond,

or a pharmaceutically acceptable salt thereof, or a prodrug thereof, or a hydrate or solvate thereof.

**3.** The compounds have the following general formula (III);

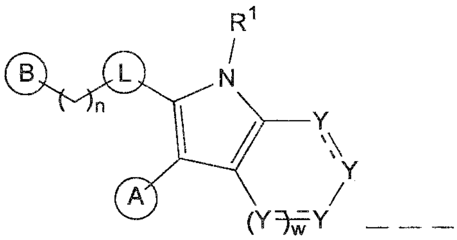
(III)

wherein, A, B, L, $R^1$ and Y are same meaning as defined in the above,

n and w represent 0 to 5,

---- represents a single bond or a double bond,

or a pharmaceutically acceptable salt thereof, or a prodrug thereof, or a hydrate or solvate thereof.

**4.** The compounds have the following general formula (IV);

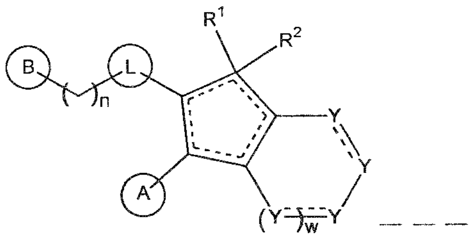
(IV)

wherein, A, B, L, $R^1$, $R^2$ and Y are same meaning as defined in the above,
n and w represent 0 to 5,
---- represents a single bond or a double bond,
or a pharmaceutically acceptable salt thereof, or a prodrug thereof, or a hydrate or solvate thereof.

**5.** The compounds as claimed in 1 -- 4, wherein, A is the substituent represented by the following general formula;

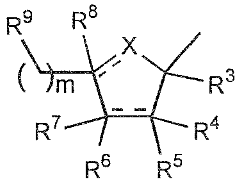

wherein,
X represents O,
---- represents a single bond or a double bond,
$R^3$, $R^4$, $R^6$, $R^7$ and $R^8$ may be either the same or different and represent hydrogen atom, hydroxyl group, amino group, mercapto group, $-(CH_2)_u$-lower alkoxy group having one to five carbon atoms, halogen atom, lower haloalkyl group having one to five carbon atoms, lower alkyl group having one to five carbon atoms, lower alkenyl group having two to five carbon atoms or lower alkynyl group having two to five carbon atoms,
u represents 0 to 5,
$R^5$ represents hydroxy group, halogen atom, lower haloalkyl group having one to five carbon atoms, lower alkyl group having one to five carbon atoms, lower alkenyl group having two to five carbon atoms or lower alkynyl group having two to five carbon atoms,
$R^9$ represents hydroxy group or halogen atom,
$R^3$, $R^4$, $R^5$, $R^6$, $R^7$, $R^8$ and $R^9$ may form the ring,
or a pharmaceutically acceptable salt thereof, or a prodrug thereof, or a hydrate or solvate thereof.

**6.** The compounds as claimed in 1 - 4, wherein, A is the substituent represented by the following general formula;

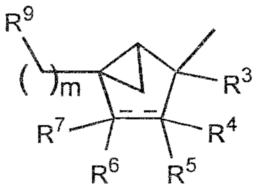

wherein,
---- represents a single bond or a double bond,
$R^3$, $R^4$, $R^5$, $R^6$ and $R^7$ may be either the same or different and represent hydrogen atom, hydroxy group, amino group, mercapto group, $-(CH_2)_u$-lower alkoxy group having one to five carbon atoms, halogen atom, lower haloalkyl group having one to five carbon atoms, lower alkyl group having one to five carbon atoms, lower alkenyl group having two to five carbon atoms or lower alkynyl group having two to five carbon atoms,
$R^9$ represents hydroxy group or halogen atom,
u represents 0 to 5,
$R^3$, $R^4$, $R^5$, $R^6$, $R^7$ and $R^9$ may optionally formed the ring,
or a pharmaceutically acceptable salt thereof, or a prodrug thereof, or a hydrate or solvate thereof.

**7.** The compoumds as claimed in 1 to 6, wherein, A is the five-membered ring that can take the dominant Northern-lock comformation,
or a pharmaceutically acceptable salt thereof, or a prodrug thereof, or a hydrate or solvate thereof.

**8.** The compoumd as claimed in 1 to 7, wherein, L is a N-$R^1$ and $R^1$ is a hydrogen atom,
or a pharmaceutically acceptable salt thereof, or a prodrug thereof, or a hydrate or solvate thereof.

**9.** The pharmaceutical composition comprising as an active ingredient a compound as claimed in 1 to 8, or a pharmaceutically acceptable salt thereof, or a prodrug thereof, or a hydrate or solvate thereof.

**10.** The pharmaceutical composition as claimed in 9 for the prevention or treatment of a disease associated with an abnormality of plasma uric acid level.

**11.** The pharmaceutical composition as claimed in claim 10, wherein, the disease is a disease selected from a group consisting of gout, hyperuricemia, urinary lithiasis, hyperuricemic nephropathy and acute uric acid nephropathy.

**12.** The pharmaceutical composition as claimed in 9 to 11 comprising in combination as an active ingredient at least one agent selected from a group consisting of colchicine, a nonsteroidal anti-inflammatory agent, a steroid, a uric acid synthesis inhibitor, a uricosuric drug, a urinary alkalinizer and a uric acid oxidase.

**13.** The pharmaceutical composition as claimed in 9 to 11 comprising in combination as an active ingredient at least one metabolic syndrome agent selected from a group consisting of an antihypertensive drug, an antihyperglycemic drug, an antiobestic drug, a nucleic acid metabolism inhibitor, a diuretic, an antituberculous drug, an anti-inflammatory drug, an antihyperlipidemic drug, an antiasthmatic drug, a cholesterol synthesis inhibitor, a cholesterol absorption inhibitor, an immunosuppressive drug, an agent for abnormality of intravascular pressure, an ischemic heart disease agent, a β-3 adrenoceptor agonist, a cannabinoid agonist or antagonist, a neuropeptide Y5 receptor antagonist , an apo-B/MTP inhibitor, a 11-β hydroxysteroid dehydrogenase-1 inhibitor, a peptidc$YY_{3\text{-}36}$ agonist, a MCR4 agonist, a CCK-A agonist, a monoamine reuptake inhibitor, a sympathetic agent, a dopamine agonist, a melanocyte-stimulating hormone receptor agonist, a leptin receptor agonist, a galanin receptor antagonist, a thyroid stimulator, a glucocorticoid receptor antagonist, an orexin receptor antagonist, an epiandrosterone dehydrogenase agent, a H-3 receptor antagonist, a lipase inhibitor, a PPAR ligand modulator, an insulinotropic agent, a sulfonylurea, a α-glucosidase inhibitor, an insulin-sensitizing drug, a non-thiazolidinone compound, a protein tyrosine phosphatase-B inhibitor, a dipeptidyl peptidase-IV inhibitor, a glucagon antagonist, a HMG-CoA reductase inhibitor, a sterol ester, an ACAT inhibitor, a bile acid reuptake inhibitor, a microsome triglyceride transporter inhibitor, a fibric acid derivatives, a β-blocker, an ACE inhibitor, a calcium channel blocker, a renin inhibitor, an AT-1 receptor antagonist, an ET receptor antagonist and an AII receptor antagonist.

**Amended claims under Art. 19.1 PCT**

**1.** . The compounds have the following general formula (I);

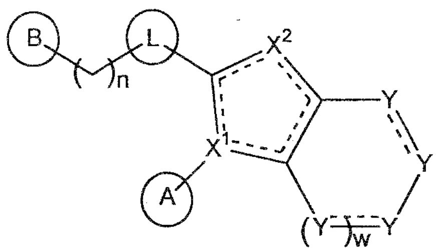

--- (I)

wherein,
n and w represent 0 to 5,
---- represents a single bond or a double bond,
$X^1$ represents N or C, provided that in the case where $X^1$ is N, $X^2$ represents C-R and in the case where $X^1$ is C, $X^2$ represents N-$R^1$, $CR^1R^2$, O or S,
wherein, R represents hydrogen atom, lower alkyl group having one to five carbon atoms, lower alkyl group including cycloalkyl ring having three to five carbon atoms, lower alkenyl group having two to five carbon atoms, lower alkynyl group having two to five carbon atoms, lower haloalkyl group having one to five carbon atoms, lower alkoxyalkyl group having two to five carbon atoms, lower hydroxyalkyl group having one to five carbon atoms, lower alkoxycarbonyl group having two to five carbon atoms, lower aminoalkyl group having one to five carbon atoms, hydroxy group, amino group, mercapto group, carboxyl group, carbamoyl group, alkanoyl group, cyano group or halogen group, $R^1$ and $R^2$ may be either the same or different and represent hydrogen atom, lower alkyl group having one

to five carbon atoms or lower haloalkyl group having two to five carbon atoms,
L represents N-$R^1$, $CR^1R^2$, lower alkenylene group having two to five carbon atoms, lower alkynylene group having two to five carbon atoms or $S(O)_v$, wherein, $R^1$ and $R^2$ are same meaning as defined in the above, v represents 0 to 2,
Y's may be either the same or different and represent $CH_2$, CH, O, N, S, N-$R^1$, CH-$NH_2$, CH-OH, CH-SH, CH-halogen atom, C-$NH_2$, C-OH, C-SH or C-halogen atom,
A represents the following general formula;

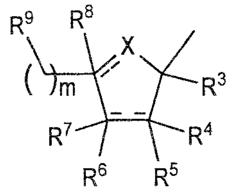

or

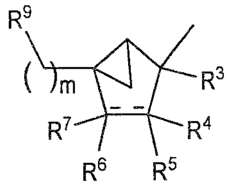

wherein,
m represents 0 to 1,
---- represents a single bond or a double bond,
X represents CH, $CH_2$, O, $S(O)_v$, N-$R^1$, $CF_2$ or -(=$CH_2$)-,
$R^3$, $R^4$, $R^5$, $R^6$, $R^7$, $R^8$ and $R^9$ may be either the same or different and represent hydrogen atom, hydroxyl group, lower alkylamino group having one to five carbon atoms, mercapto group , -$(CH_2)_u$-lower alkoxy group having one to five carbon atoms, halogen atom, lower haloalkyl group having one to five carbon atoms , lower alkyl group having one to five carbon atoms , lower alkyl group including cycloalkyl ring having three to five carbon atoms, lower alkenyl group having two to five carbon atoms or lower alkynyl group having two to five carbon atoms, $R^3$, $R^4$, $R^5$, $R^6$, $R^7$, $R^8$ and $R^9$ optionally formed cycle or heterocyclo ring,
u represents 0 to 5,
however, in the general formula (I), in the case where $X^1$ is N, $X^2$ is C-CN, X is O,
---- is a single bond and $R^5$, $R^6$ and $R^9$ are hydroxyl group, $R^4$ is not hydrogen atom ,
B represents the following general formula;

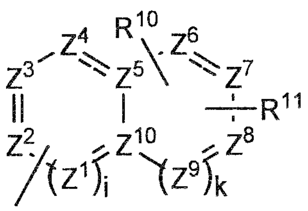

or

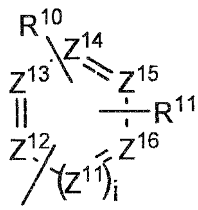

wherein,
i and k represent 0 to 5,
---- represents a single bond or a double bond,
$Z^1$, $Z^2$, $Z^3$, $Z^4$, $Z^5$, $Z^6$, $Z^7$, $Z^8$, $Z^9$, $Z^{10}$, $Z^{11}$, $Z^{12}$, $Z^{13}$, $Z^{14}$, $Z^{15}$ and $Z^{16}$ may be either the same or different and represent CH, $CH_2$, O, S, N or NH,
$R^{10}$ and $R^{11}$ may be either the same or different and represent hydrogen atom, halogen atom, lower alkyl group having one to five carbon atoms, optionally substituted cycloalkyl group having three to eight carbon atoms, optionally substituted heterocycloalkyl group, lower haloalkyl group having one to five carbon atoms, lower alkenyl group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, nitro group, cyano group, optionally substituted mono or bicyclic aromatic ring having five to ten carbon atoms, optionally substituted mono or bicyclic heteroaromatic ring having one to ten carbon atoms, optionally substituted benzyl group, optionally substituted amino group which can be optionally formed a quaternary ammonium compound, -CON$R^{12}R^{13}$, -NHCO$R^{15}$, -$(CH_2)_o$-$R^{15}$-$(CH_2)_p$-$R^{16}$, -lower alkenylene group having two to five carbon atoms-$R^{15}$, -lower alkynylene group having two to five carbon atoms-$R^{15}$, -$(CH_2)_o$-cycloalkyl group having three to eight carbon atoms-$(CH_2)_p$-$R^{15}$, -$(CH_2)_o$-heterocycloalkyl group-$(CH_2)_p$-$R^{15}$, -$(CH_2)_o$-NHC(=NH)-$NH_2$, -$(CH_2)_o$-C(=NH)-$NH_2$, -$(CH_2)_o$-D-$(CH_2)_t$-E-$(CH_2)_p$-$R^{15}$ or a group represented by the following general formula;

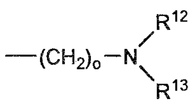 , 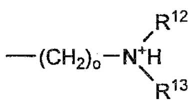 , 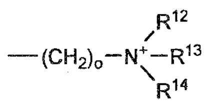 , 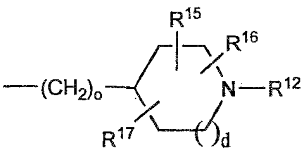 ,

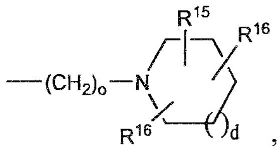 , 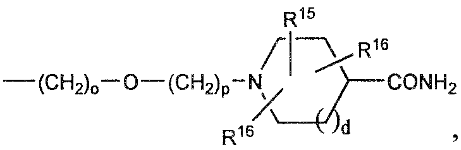 , 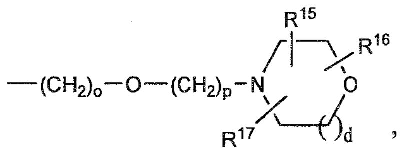 ,

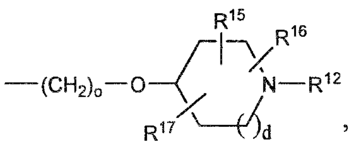 , 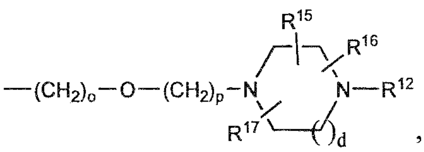 , 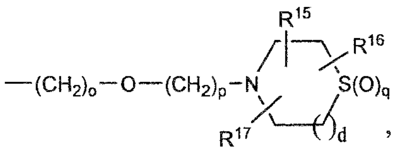 ,

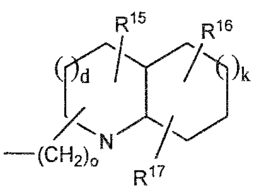 or 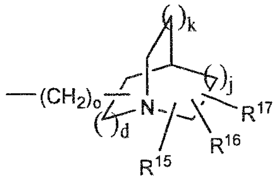

wherein,
o and p may be either the same or different and represent 0 to 15,
t represents 1 to 5,
q represents 0 to 2,
d, k and j represent 0 to 5,
D and E may be either the same or different and represent single bond(-), $-CONR^{12}-$, $-NR^{12}CO-$, -O-, $-NR^{12}-$, $-S(O)_q-$, $-N R^{12} S(O)_q-$, $-S(O)_qN R^{12}-$ or -NHC(=O)NH-,
$R^{12}$, $R^{13}$ and $R^{14}$ may be either the same or different and represent hydrogen atom, carboxyl group, lower alkyl group having one to five carbon atoms or lower hydroxy alkyl group having one to five carbon atoms and optionally formed the ring,
$R^{15}$, $R^{16}$ and $R^{17}$ represent hydrogen atom, halogen atom, lower alkyl group having one to five carbon atoms, optionally substituted cycloalkyl group having three to eight carbon atoms, optionally substituted heterocycloalkyl group, lower haloalkynylene group having two to five carbon atoms, lower alkenylene group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, sulfonic acid group, phosphoric acid group, nitro group, cyano group, aminosulfonyl group, optionally substituted mono or bicyclic aromatic ring having five to ten carbon atoms, optionally substituted mono or bicyclic heteroaromatic ring having one to ten carbon atoms, optionally substituted benzyl group, $-S(O)_q$-lower alkyl group having one to five carbon atoms, $-CONR^{18}R^{19}$, optionally substituted amino group which can be optionally formed a quaternary ammonium compound, amino acid residue, sugar residue or a group represented by the following general formula;

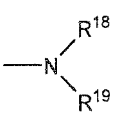 , 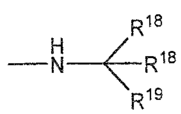 ,

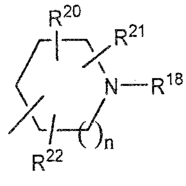 , 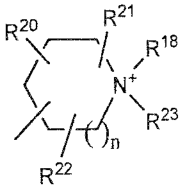 or 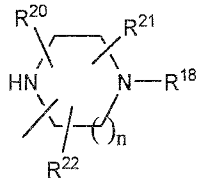

wherein,
$R^{18}$ and $R^{19}$ represent hydrogen atom, lower alkyl group having one to five carbon atoms, lower alkylcarbonyl group having two to five carbon atoms, lower alkylsulfonyl group having one to five carbon atoms, optionally substituted benzenesulfonyl group, lower alkoxycarbonyl group having two to five carbon atoms, lower hydroxyalkyl group having one to five carbon atoms, hydroxyethoxy group, alkoxyethoxy group, optionally substituted aminoethoxy group or $-(CH_2)_o-CONH_2$,
$R^{20}$, $R^{21}$ and $R^{22}$ represent hydrogen atom, halogen atom, lower alkyl group having one to five carbon atoms, optionally substituted cycloalkyl group having three to eight carbon atoms, optionally substituted heterocycloalkyl group, lower haloalkynylene group having two to five carbon atoms, lower alkenylene group having two to five carbon atoms, lower alkynyl group having two to five carbon atoms, hydroxy group, lower alkoxy group having one to five carbon atoms, lower haloalkoxy group having one to five carbon atoms, lower alkoxycarbonyl group having two to five carbon atoms, carboxyl group, nitro group, cyano group, aminosulfonyl group, optionally substituted mono or bicyclic aromatic ring having five to ten carbon atoms, optionally substituted mono or bicyclic heteroaromatic ring having one to ten carbon atoms, optionally substituted benzyl group, $-S(O)_q$-lower alkyl group having one to five carbon atoms, optionally substituted amino group which can be optionally formed a quaternary ammonium compound,
$R^{23}$ represents lower alkyl group having one to five carbon atoms,
or a pharmaceutically acceptable salt thereof, or a prodrug thereof, or a hydrate or solvate thereof.

**2.** . The compounds have the following general formula (II);

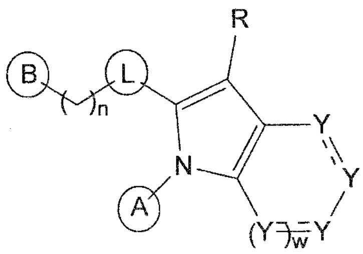 --- (II)

wherein, A, B, L, R and Y are same meaning as defined in the above,
n and w represent 0 to 5,
---- represents a single bond or a double bond,
or a pharmaceutically acceptable salt thereof, or a prodrug thereof, or a hydrate or solvate thereof.

**3.** . The compounds have the following general formula (III);

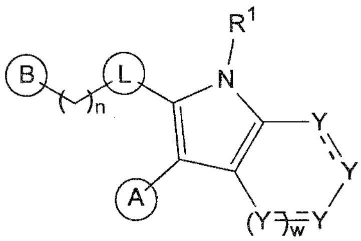 --- (III)

wherein, A, B, L, $R^1$ and Y are same meaning as defined in the above,
n and w represent 0 to 5,
---- represents a single bond or a double bond,
or a pharmaceutically acceptable salt thereof, or a prodrug thereof, or a hydrate or solvate thereof.

**4.** . The compounds have the following general formula (IV);

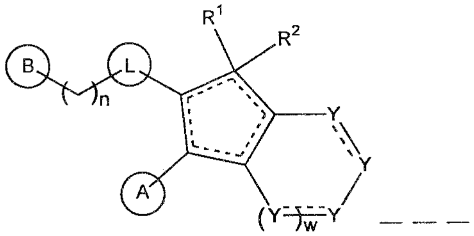

(IV)

wherein, A, B, L, $R^1$, $R^2$ and Y are same meaning as defined in the above,
n and w represent 0 to 5,
---- represents a single bond or a double bond,
or a pharmaceutically acceptable salt thereof, or a prodrug thereof, or a hydrate or solvate thereof.

**5.** . The compounds as claimed in 1 - 4, wherein, A is the substituent represented by the following general formula;

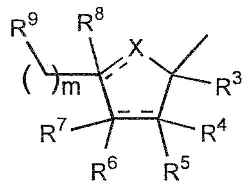

wherein,
X represents O,
---- represents a single bond or a double bond,
$R^3$, $R^4$, $R^6$, $R^7$ and $R^8$ may be either the same or different and represent hydrogen atom, hydroxyl group, amino group, mercapto group, $-(CH_2)_u$-lower alkoxy group having one to five carbon atoms, halogen atom, lower haloalkyl group having one to five carbon atoms, lower alkyl group having one to five carbon atoms, lower alkenyl group having two to five carbon atoms or lower alkynyl group having two to five carbon atoms,
u represents 0 to 5,
$R^5$ represents hydroxy group, halogen atom, lower haloalkyl group having one to five carbon atoms, lower alkyl group having one to five carbon atoms, lower alkenyl group having two to five carbon atoms or lower alkynyl group having two to five carbon atoms,
$R^9$ represents hydroxy group or halogen atom,
$R^3$, $R^4$, $R^5$, $R^6$, $R^7$, $R^8$ and $R^9$ may form the ring,
or a pharmaceutically acceptable salt thereof, or a prodrug thereof, or a hydrate or solvate thereof.

**6.** . The compounds as claimed in 1 - 4, wherein, A is the substituent represented by the following general formula;

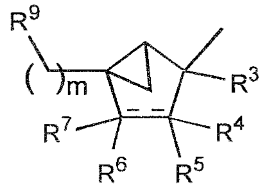

wherein,
---- represents a single bond or a double bond,
$R^3$, $R^4$, $R^5$, $R^6$ and $R^7$ may be either the same or different and represent hydrogen atom, hydroxy group, amino group, mercapto group, $-(CH_2)_u$-lower alkoxy group having one to five carbon atoms, halogen atom, lower haloalkyl group having one to five carbon atoms, lower alkyl group having one to five carbon atoms, lower alkenyl group having two to five carbon atoms or lower alkynyl group having two to five carbon atoms,
$R^9$ represents hydroxy group or halogen atom,
u represents 0 to 5,
$R^3$, $R^4$, $R^5$, $R^6$, $R^7$ and $R^9$ may optionally formed the ring,
or a pharmaceutically acceptable salt thereof, or a prodrug thereof, or a hydrate or solvate thereof.

**7.** . The compoumds as claimed in 1 to 6, wherein, A is the five-membered ring that can take the dominant Northern-lock comformation,
or a pharmaceutically acceptable salt thereof, or a prodrug thereof, or a hydrate or solvate thereof.

**8.** . The compoumd as claimed in 1 to 7, wherein, L is a N-$R^1$ and $R^1$ is a hydrogen atom,
or a pharmaceutically acceptable salt thereof, or a prodrug thereof, or a hydrate or solvate thereof.

**9.** . The pharmaceutical composition comprising as an active ingredient a compound as claimed in 1 to 8,
or a pharmaceutically acceptable salt thereof, or a prodrug thereof, or a hydrate or solvate thereof.

**10.** . The pharmaceutical composition as claimed in 9 for the prevention or treatment of a disease associated with an abnormality of plasma uric acid level.

**11.** . The pharmaceutical composition as claimed in claim 10, wherein, the disease is a disease selected from a group consisting of gout, hyperuricemia, urinary lithiasis, hyperuricemic nephropathy and acute uric acid nephropathy.

**12.** . The pharmaceutical composition as claimed in 9 to 11 comprising in combination as an active ingredient at least one agent selected from a group consisting of colchicine, a nonsteroidal anti-inflammatory agent, a steroid, a uric acid synthesis inhibitor, a uricosuric drug, a urinary alkalinizer and a uric acid oxidase.

**13.** . The pharmaceutical composition as claimed in 9 to 11 comprising in combination as an active ingredient at least one metabolic syndrome agent selected from a group consisting of an antihypertensive drug, an antihyperglycemic drug, an antiobestic drug, a nucleic acid metabolism inhibitor, a diuretic, an antituberculous drug, an anti-inflammatory drug, an antihyperlipidemic drug, an antiasthmatic drug, a cholesterol synthesis inhibitor, a cholesterol absorption inhibitor, an immunosuppressive drug, an agent for abnormality of intravascular pressure, an ischemic heart disease agent, a β-3 adrenoceptor agonist, a cannabinoid agonist or antagonist, a neuropeptide Y5 receptor antagonist , an apo-B/MTP inhibitor, a 11-β hydroxysteroid dehydrogenase-1 inhibitor, a peptide$YY_{3-36}$ agonist, a MCR4 agonist, a CCK-A agonist, a monoamine reuptake inhibitor, a sympathetic agent, a dopamine agonist, a melanocyte-stimulating hormone receptor agonist, a leptin receptor agonist, a galanin receptor antagonist, a thyroid stimulator, a glucocorticoid receptor antagonist, an orexin receptor antagonist, an epiandrosterone dehydrogenase agent, a H-3 receptor antagonist, a lipase inhibitor, a PPAR ligand modulator, an insulinotropic agent, a sulfonylurea, a α-glucosidase inhibitor, an insulin-sensitizing drug, a non-thiazolidinone compound, a protein tyrosine phosphatase-B inhibitor, a dipeptidyl peptidase-IV inhibitor, a glucagon antagonist, a HMG-CoA reductase inhibitor, a sterol ester, an ACAT inhibitor, a bile acid reuptake inhibitor, a microsome triglyceride transporter inhibitor, a fibric acid derivatives, a β-blocker, an ACE inhibitor, a calcium channel blocker, a renin inhibitor, an AT-1 receptor antagonist, an ET receptor antagonist and an AII receptor antagonist.

Figure 1

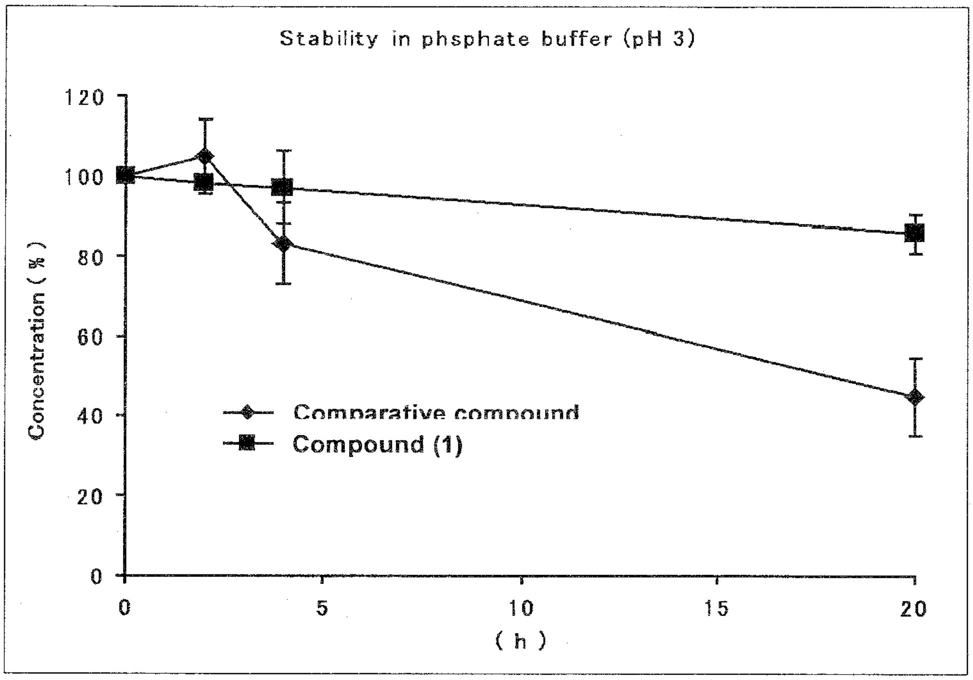
Stability in phsphate buffer (pH 3)
120
100
80
60
40
20
0
Concentration ( % )
Comparative compound
Compound (1)
0
5
10
15
20
( h )

## INTERNATIONAL SEARCH REPORT

International application No.
PCT/JP2009/065376

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07H19/14, A61K31/517, A61K31/519, A61K31/7042, A61K45/00, A61K45/06, A61P3/06, A61P3/10, A61P9/10, A61P13/02, A61P13/04, A61P19/06, A61P43/00, C07D239/70

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2007-277203 A (Kissei Pharmaceutical Co., Ltd.), 25 October 2007 (25.10.2007), entire text; particularly, examples (Family: none) | 1,2,5,7-13<br>1,2,5,7-13<br>3,4,6 |
| X | SEELA, F., et al., Regioselectivity of the Mannich reaction on pyrrolo[2,3-d]pyrimidine nucleosides related to 7-deaza-2'-deoxyadenosine or 7-deaza-2'-deoxyguanosine, Synthesis, (9), pp.1067-1072 (1997), entire text | 1,2,5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 November, 2009 (09.11.09) | 24 November, 2009 (24.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

INTERNATIONAL SEARCH REPORT

International application No.
PCT/JP2009/065376

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 2005/063788 A1 (Kissei Pharmaceutical Co., Ltd.),<br>14 July 2005 (14.07.2005),<br>claims; examples<br>& EP 1698635 A1 & US 2008/038242 A1<br>& US 7547680 B2 & TW 200530224 A | 1,2,5,7-13<br>3,4,6 |
| Y<br>A | WO 2006/030803 A1 (Kissei Pharmaceutical Co., Ltd.),<br>23 March 2006 (23.03.2006),<br>claims; examples<br>& EP 1813623 A1 & US 2007/179115 A1<br>& TW 200626609 A | 1,2,5,7-13<br>3,4,6 |
| Y<br>A | WO 2006/115137 A1 (Kissei Pharmaceutical Co., Ltd.),<br>02 November 2006 (02.11.2006),<br>claims; examples<br>(Family: none) | 1,2,5,7-13<br>3,4,6 |

Form PCT/ISA/210 (continuation of second sheet) (April 2007)

**INTERNATIONAL SEARCH REPORT**

International application No.
PCT/JP2009/065376

Continuation of A. CLASSIFICATION OF SUBJECT MATTER
(International Patent Classification (IPC))

*C07H19/14*(2006.01)i, *A61K31/517*(2006.01)i, *A61K31/519*(2006.01)i, *A61K31/7042*(2006.01)i, *A61K45/00*(2006.01)i, *A61K45/06*(2006.01)i, *A61P3/06*(2006.01)i, *A61P3/10*(2006.01)i, *A61P9/10*(2006.01)i, *A61P13/02*(2006.01)i, *A61P13/04*(2006.01)i, *A61P19/06*(2006.01)i, *A61P43/00*(2006.01)i, *C07D239/70*(2006.01)i

(According to International Patent Classification (IPC) or to both national classification and IPC)

Form PCT/ISA/210 (extra sheet) (April 2007)

INTERNATIONAL SEARCH REPORT

International application No.
PCT/JP2009/065376

Claims 1 to 13

In claim 1, general formula (I) is presented with respect to the structure of the compound and various chemical structures are cited as alternative forms.

Although $CH_2$, CH or the like are cited as Y, the description shows that products having N and C=O that are not included in the scope of the alternatives of Y were also produced in practice. Accordingly, the disclosure makes the invention in the aforesaid claim unclear.

Although "prodrug" is described in the aforesaid claim, it is not clearly disclosed therein how chemically modified compounds the prodrug means. Thus, the invention in the aforesaid claim is unclear.

Although there are a large number of compounds having such structures as satisfying the requirements needed for the compounds of the invention in the aforesaid claim that involve alternative chemical structures as described above, the structures common to these compounds are merely the fused ring in general formula (I) and the five-membered ring common to the general formulae relating to A. However, these structures are publicly known (see, for example, JP 2007-277203 A) and so are the functions of the same. Such being the case, a single comprehensive inventive concept cannot be clearly understood merely on the basis of these structures, which makes the invention in the aforesaid claim unclear.

Since the invention in claim 1 involves such a large number of compounds as described above and the structures thereof widely vary, it should be described in the description that compounds having various structures were produced in practice in order to disclose the whole range of the compounds in the description and to conclude that these compounds can be produced.

In the present case, although some compounds were produced in practice and the physiological activities thereof were discussed, structural formulae are merely represented for many of other compounds or even no structural formula is presented in some cases. By taking the description relating to the compounds that were produced in practice into consideration, it does not appear that there is any special reason by which the invention can be extended or generalized to the whole range of the other compounds. Such being the case, it cannot be considered that the description discloses the whole range of the invention in claim 1. Also, it cannot be considered that the description discloses the invention in a manner sufficiently clear and complete for the invention to be carried out by a person skilled in the art (PCT Articles 5 and 6).

These points apply to other claims.

Since the present invention is not sufficiently disclosed as discussed above, the search was conducted on prior art within a reasonable scope on the basis of the description in establishing the international search report.

(continued to next extra sheet)

Form PCT/ISA/210 (extra sheet) (April 2007)

INTERNATIONAL SEARCH REPORT

International application No.
PCT/JP2009/065376

Claims 2 to 13

In claims 2 to 4, various symbols used in the general formulae are defined as "having the same meaning as defined above". However, these claims depend on no other claim and, therefore, the definitions are not clearly specified, which makes the inventions in these claims unclear.

The same applies to other claims depending on claims 2 to 4.

Page 3 in description

Although "International Publication No. 2007/277203 pamphlet" is cited as "Patent Document 4" in the aforesaid page, there is no international publication corresponds thereto. Thus, the citation is regarded as an obvious error.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200506378 A
- WO 2006030803 A
- WO 2006115137 A
- WO 2007277203 A
- WO 2004002422 A
- WO 03068162 A


### Non-patent literature cited in the description


- **Ho-Chul Shin ; Jin-Suk Kim ; Balvinder Singh Vig ; Xueqin Song ; John Charles Drach ; Gordon Lewis Amidon.** *Biol. Pharm. Bull.,* vol. 29 (2), 247
- **C. Altona ; M. J. Sundaralingham.** *J. Am. Chem. Soc.,* 1972, vol. 94, 8205
- **Jonathan K. Watts ; Kashinath Sadalapure ; Niloufar Choubdar ; B. Mario Pinto ; Masad J. Damha.** *J. Org. Chem.,* 2006, vol. 71, 921
- **Victor E. Marquez ; Maqbool A. Siddiqui ; Abdallah Ezzitouni ; Pamela Russ ; Jianying Wang ; Richard W. Wagner ; Mark D. Matteucci.** *J. Med. Chem.,* 1996, vol. 39, 3739
- Handbook of Pharmaceutical Salts. WILEY-VCH, 2002
- Protective Groups in Organic Synthesis. WILEY-Interscience, 1999
- *J. Org. Chem.,* 1981, vol. 46 (13), 2891-2823
- *Chem. Pharm. Bull.,* 1977, vol. 25 (4), 575-578
- *Tetrahedron,* 1970, vol. 26, 4251-4259
- *Tetrahedron Lett.,* 1999, vol. 40, 3309-3312
- *J. Org. Chem.,* 1999, vol. 64, 4172-4178
- *Bioorg. Med. Chem. Lett.,* 2006, vol. 16, 285-287
- *J. Med. Chem.,* 2001, vol. 44, 3985-3993
- *Tetrahedron Lett.,* 1997, vol. 38, 4207-4210
- *Tetrahedron Lett.,* 2005, vol. 46, 8913-8915
- *Tetrahedron Lett.,* 2004, 8981-8982
- *J. Org. Chem.,* 2004, vol. 69, 3993-3996
- *J. Med. Chem.,* 2006, vol. 49, 1140-1148
- *Org. Lett.,* 2001, vol. 3, 597-599
- *NUCLEOSIDE&NUCLEOTIDE,* 1994, vol. 13, 2035-2050
- *J. Org. Chem.,* 1997, vol. 62, 1754-1759
- *J. Org. Chem.,* 2006, vol. 71, 4018-4020
- *J. Med. Chem.,* 2006, vol. 49, 273-281
- *J. Org. Chem.,* 2006, vol. 71, 921-925